(19) Europäisches Patentamt / European Patent Office / Office européen des brevets

(11) **EP 1 185 708 B1**

(12) **FASCICULE DE BREVET EUROPEEN**

(45) Date de publication et mention
de la délivrance du brevet:
**10.09.2008 Bulletin 2008/37**

(51) Int Cl.:
***C12Q 1/68*** *(2006.01)* ***C07K 14/415*** *(2006.01)*

(21) Numéro de dépôt: **00951636.0**

(86) Numéro de dépôt international:
**PCT/FR2000/001723**

(22) Date de dépôt: **21.06.2000**

(87) Numéro de publication internationale:
**WO 2000/079001 (28.12.2000 Gazette 2000/52)**

(54) **MOYENS POUR L'IDENTIFICATION D'UNE NOUVELLE CLASSE DE GENES DE RESISTANCE AU VIRUS DE LA PANACHURE JAUNE DU RIZ ET DU LOCUS D'UN GENE MAJEUR DE LA RESISTANCE AU VIRUS, ET LEURS APPLICATIONS**

MITTEL ZUR IDENTIFIZIERUNG EINER NEUEN, FÜR DIE RESISTENZ GEGENÜBER DEM RICE YELLOW MOTTLE VIRUS VERANTWORTLICHEN GENKLASSE UND ZUR IDENTIFIZIERUNG DES GENORTES EINES FUR DIE RESISTENZ GEGENÜBER DEM RICE YELLOW MOTTLE VIRUS WICHTIGEN GENES UND DEREN ANWENDUNG

MEANS FOR IDENTIFYING A NOVEL CLASS OF GENES RESISTANT TO THE RICE YELLOW MOTTLE VIRUS AND THE LOCUS OF A MAJOR GENE OF RESISTANCE TO THE VIRUS, AND THEIR APPLICATIONS

(84) Etats contractants désignés:
**AT BE CH CY DE DK ES FI FR GB GR IE IT LI LU MC NL PT SE**

(30) Priorité: **21.06.1999 FR 9907831**

(43) Date de publication de la demande:
**13.03.2002 Bulletin 2002/11**

(73) Titulaire: **Institut de Recherche pour le Développement ( IRD)**
**75480 Paris Cedex 10 (FR)**

(72) Inventeurs:
• **BRUGIDOU, Christophe**
**F-34570 Pignan (FR)**
• **BRIZARD, Jean-Paul**
**F-34380 Saint Martin de Londres (FR)**
• **GHESQUIERE, Alain**
**F-34000 Montpellier (FR)**

(74) Mandataire: **Peaucelle, Chantal et al**
**Cabinet Armengaud Aîné**
**3, Avenue Bugeaud**
**75116 Paris (FR)**

(56) Documents cités:
**WO-A-98/30721 US-A- 5 898 097**

• **BONNEAU CAROLINE ET AL: "Expression of the rice yellow mottle virus P1 protein in vitro and in vivo and its involvement in virus spread." VIROLOGY, vol. 244, no. 1, 25 avril 1998 (1998-04-25), pages 79-86, XP002149717 ISSN: 0042-6822**
• **OPALKA NATACHA ET AL: "Movement of rice yellow mottle virus between xylem cells through pit membranes." PROCEEDINGS OF THE NATIONAL ACADEMY OF SCIENCES OF THE UNITED STATES, vol. 95, no. 6, 17 mars 1998 (1998-03-17), pages 3323-3328, XP002149718 March 17, 1998 ISSN: 0027-8424**
• **ALBAR, L. (1) ET AL: "Genetic basis and mapping of the resistance to rice yellow mottle virus: I. QTLs identification and relationship between resistance and plant morphology." THEORETICAL AND APPLIED GENETICS, (NOV., 1998) VOL. 97, NO. 7, PP. 1145-1154., XP000892947**
• **PRESSOIR, G. ET AL: "Genetic basis and mapping of the resistance to rice yellow mottle virus: II. Evidence of a complementary epistasis between two QTLs." THEORETICAL AND APPLIED GENETICS, (NOV., 1998) VOL. 97, NO. 7, PP. 1155-1161., XP000892950**

EP 1 185 708 B1

- **DATABASE EMBL [en ligne] embl; Database entry/Accession number AZ132900, 10 juin 2000 (2000-06-10) WING ET AL : "A BAC sequencing framework to sequence the rice genome" XP002149653**

Remarques:

Le dossier contient des informations techniques présentées postérieurement au dépôt de la demande et ne figurant pas dans le présent fascicule.

LISTE DE SEQUENCES

<110> I.R.D.

<120> Moyens pour l'identification d'une nouvelle classe de gènes de résistance au virus de la panachure jaune du riz et du locus d'un gène majeur de la résistance au virus, et leurs applications.

<130> 59782-1158

<140>
<141>

<150> 9907831
<151> 1999-06-21

<160> 12

<170> PatentIn Ver. 2.1

<210> 1
<211> 16
<212> ADN
<213> Séquence artificielle

<220>
<223> Description de la séquence artificielle:Nucléotide

<400> 1
gactgcgtac caattc          16

<210> 2
<211> 16
<212> ADN
<213> Séquence artificielle

<220>
<223> Description de la séquence artificielle:Nucléotide

<400> 2
gatgagtcct gagtaa          16

<210> 3
<211> 472
<212> ADN
<213> Séquence artificielle

<220>
<223> Description de la séquence artificielle:Nucléotide

<400> 3

```
cgtgcttgct tatagcacta caggagaagg aaggggaaca caacagccat ggcgagcgaa  60
ggttcaacgt cggagaaaca ggctgcgacg ggcagcaagg tgccggcggc ggatcggagg 120
aaggaaaagg aggaaatcga agttatgctg gaggggcttg acctaagggc agatgaggag 180
gaggatgtgg aattggagga agatctagag gagcttgagg cagatgcaag atggctagcc 240
ctagccacag ttcatacgaa gcgatcgttt agtcaagggg ctttctttgg gagtatgcgc 300
tcagcatgga actgcgcgaa agaagtagat ttcagagcaa tgaaagacaa tctgttctcg 360
atccaattca attgtttggg ggattgggaa cgagttatga atgaaggtcc atggaccttt 420
cgaggatgtt cggtgctcct cgcagaatat gatggctggt ccaagattga at           472
```

<210> 4
<211> 21
<212> ADN
<213> Séquence artificielle

<220>
<223> Description de la séquence artificielle:Nucléotide

<400> 4
aggaagggga acacaacagc c           21

<210> 5
<211> 21
<212> ADN
<213> Séquence artificielle

<220>
<223> Description de la séquence artificielle:Nucléotide

<400> 5
ttatgctgga ggggcttgac c           21

<210> 6
<211> 21
<212> ADN
<213> Séquence artificielle

<220>
<223> Description de la séquence artificielle:Nucléotide

<400> 6
gcagttccat gctgagcgca t           21

<210> 7
<211> 21
<212> ADN
<213> Séquence artificielle

<220>
<223> Description de la séquence artificielle:Nucléotide

<400> 7
ccgaacatcc tcgaaaggtc c          21

<210> 8
<211> 21
<212> ADN
<213> Séquence artificielle

<220>
<223> Description de la séquence artificielle:Nucléotide

<400> 8
tcatattctg cgaggagcac c          21

<210> 9
<211> 121
<212> ADN
<213> Séquence artificielle

<220>
<223> Description de la séquence artificielle:Nucléotide

<400> 9

```
aattcacccc atgccctaag ttaggacgtt ctcagcttag tggtgtggta gctttttcta 60
ttttcctaag cacccattga agtattttgc attggaggtg gccttaggtt tgcctctgtt 120
a                                                                 121
```

<210> 10
<211> 20
<212> ADN
<213> Séquence artificielle

<220>
<223> Description de la séquence artificielle:Nucléotide

<400> 10
aacctaaggc cacctccaat          20

<210> 11
<211> 19
<212> ADN
<213> Séquence artificielle

<220>
<223> Description de la séquence artificielle:Nucléotide

<400> 11
gcaaacctaa ggccacctc          19

<210> 12
<211> 19
<212> ADN
<213> Séquence artificielle

<220>

<223> Description de la séquence artificielle:Nucléotide

<400> 12
attcacccca tgccctaag          19

**Description**

Moyens pour l'identification d'une nouvelle classe de gènes de résistance au virus de la panachure jaune du riz et du locus d'un gène majeur de la résistance au virus, et leurs applications.

Des moyens, outils et procédés pour l'identification d'une nouvelle classe de gènes de résistance au virus de la panachure jaune du riz (en abrégé RYMV pour Rice Yellow Mottle Virus) et du locus d'un gène majeur de la résistance au virus sont décrits.

Sont visés plus spécialement, en tant qu'outils, des protéines essentielles pour le cycle infectieux ainsi que des marqueurs et des amorces PCR et leurs applications à l'établissement de la cartographie physique de la résistance et au clonage du gène.

RYMV est un virus endémique en Afrique. Il présente des caractéristiques communes avec les autres sobémovirus ; à savoir un seul ARN simple brin de polarité positive non polyadénylé et de petite taille, et des particules icosahédriques de symétrie T=3 produites en très grande quantité dans la plante. Les particules virales sont aussi très présentes dans les tissus vasculaires et principalement dans les vaisseaux. Chez quelques rares variétés de l'espèce africaine de riz cultivé *Oryza glaberrima,* une résistance très élevée au RYMV a été identifiée. Mais comme les hybrides interspécifiques entre les deux espèces de riz cultivées sont extrêmement stériles, les recherches antérieures n'ont pas permis de décrire ni de bases génétiques, ni de mécanisme de cette résistance.

Les travaux des inventeurs dans ce domaine ont montré qu'une variété dénommée *Gigante,* originaire du Mozambique et identifiée par l'ADRAO, de l'espèce asiatique de riz cultivé *Oryza sativa,* manifestait les mêmes caractéristiques que celles observées chez *O. glaberrima.* Les inventeurs ont caractérisé la résistance à RYMV en mettant en évidence qu'elle est liée à un gène majeur de résistance récessif et identique chez les deux sources de résistance considérée (*O. Sativa* et *O. glaberrima*).

Cette résistance intervient au niveau du mouvement de cellule à cellule et se traduit par un blocage du virus au niveau des cellules infectées alors que la réplication du virus est normale.

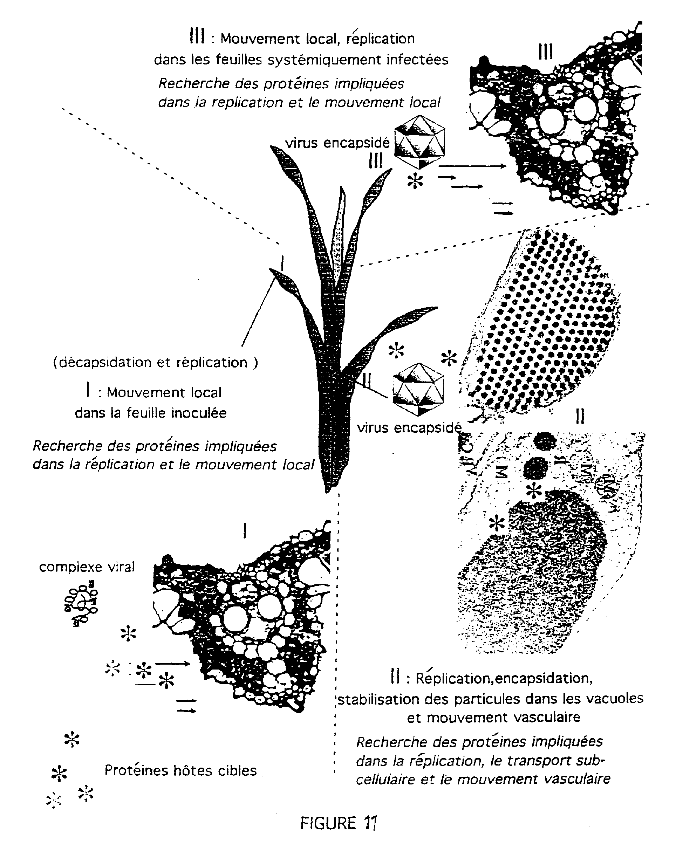

Les travaux des inventeurs sur le RYMV ont montré que ce virus bouge et se multiplie différemment au cours du cycle infectieux. Dans la feuille inoculée (I, figure 11), c'est sous forme d'un complexe ARN viral et protéines virales (protéines de capside, P1 et le cas échéant P3) qu'il se déplace localement en traversant les plasmodesmes des cellules épidermiques, des cellules du mésophylle, des cellules de la gaine périvasculaire (méstome et cellule de la gaine) pour atteindre les cellules vasculaires (parenchyme phloémien et xylémien). Il se multiplie plus particulièrement dans les cellules du parenchyme xylèmien (II, figure 11). Dans les cellules vasculaires, avant le mouvement dit de longue distance, il s'encapside et se stabilise sous forme d'une particule compacte dans la vacuole à un pH acide et grâce aux ions divalents $Ca_2^+$ (II, figure 11). L'infection systémique ne peut se faire que si un grand nombre de particules stables sont produites. Dans les feuilles systématiquement infectées, le virus sort des tissus conducteurs pour se multiplier soit dans les jeunes tissus vasculaires, soit dans les cellules du mésophylle. A ce stade de l'infection le mouvement local se fait sous forme décapsidée (complexe viral RNA et protéines) ou encapsidée toujours à travers les plasmodesmes (III, figure 11).

Lors de ces différentes étapes du cycle infectieux, le complexe viral et /ou le virion ont besoin d'être identifié et transporté par des protéines de la plante pour bouger, d'un compartiment cellulaire à un autre, d'une cellule à l'autre.

Par exemple, les protéines de plantes liées au transport du virus ou de son complexe apparaissent présenter une fonction similaire (transport), mais sont spécifiques du tissu traversé (épiderme, mésophylle, mestome, gaine périvasculaire, phloème, xylème). Lorsque ces protéines sont traduites de l'allèle sauvage, ce sont des protéines de susceptibilité qui permettent au virus de se déplacer. Au contraire, l'allèle muté apparaît conduire soit à une protéine moins fonctionnelle (résistance partielle), soit non fonctionnelle (résistance totale).

Il est donc considéré que ces protéines appartiennent à une famille de gènes dont la fonction cellulaire commune serait la reconnaissance du substrat et le transport à travers les plasmodesmes, mais sont différentes au niveau de la spécificité tissulaire (épiderme, mésophylle, mestome, gaine périvasculaire, phloème, xylème).

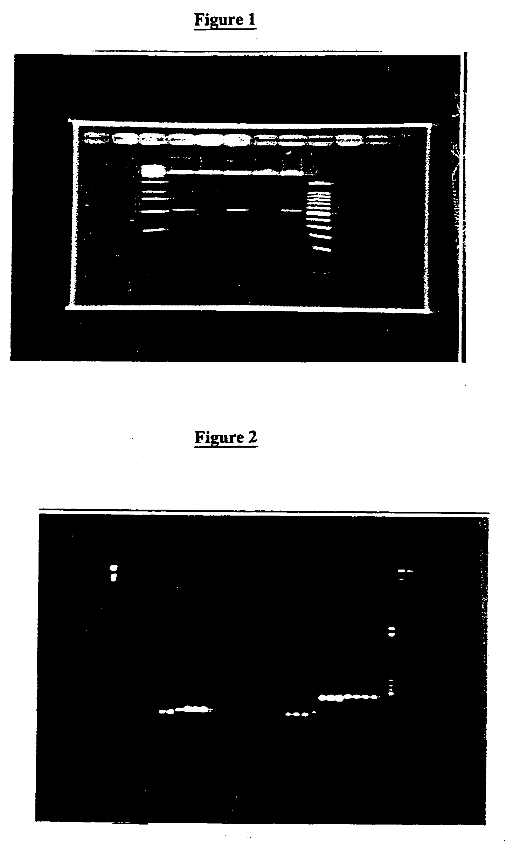

Opalka et al. (Proceedings of the National Academy of Sciences USA, 1998, 95:3323-3328) décrit (cf. résumé, figure 1 et page 3323, colonne 2, ligne 37 - page 3324, colonne 1, ligne 2) une méthode d'isolement de protéines impliquées dans le transport d'un virus pathogène.

La régulation du transport symplastique est très probablement la fonction essentielle de cette famille de gènes.

Le RYMV est aussi un virus très stable et il se présente dans la cellule sous plusieurs isoformes dont trois ont été déterminées : compacte, gonflée et intermédiaire. Ainsi, suivant le pH cellulaire (cytoplasme 7-8, vacuoles, vésicules et vaisseaux 4,5-6,5) la conformation et la charge extérieure de la particule changent. Cette charge lui permet de s'accrocher aux membranes et d'entrer dans une cellule saine par un mécanisme d'endocytose. Enfin, au niveau cellulaire, les inventeurs ont montré que ce virus s'accumule principalement dans les vacuoles. La présence *in planta* de trois isoformes, la compartimentation et l'accumulation virale dans les plantes partiellement résistantes ont ainsi permis de proposer un mécanisme original pour la tolérance au RYMV, distinct de la résistance.

La tolérance au RYMV apparaît en effet s'effectuer grâce à une accumulation dans les vacuoles. Le tonoplaste, en séparant physiquement les particules virales du compartiment cellulaire, empêcherait toute interaction dommageable pour la machinerie cellulaire. Ainsi, le virus se multiplie, s'accumule sans tuer la cellule (donc pas de symptômes).

L'association entre la cellule hôte et le virus est tel que la plante se comporte alors comme une plante réservoir.

Dans ce modèle la co-évolution entre le virus et la plante hôte a conduit le virus à s'adapter pour finalement être reconnu par la plante comme une simple protéine de réserve qui serait produite par la plante, transportée via le reticulum et l'appareil de Golgi vers la vacuole. Une invagination intense du tonoplaste (mécanisme d'autophagie) pourrait aussi permettre au virus produit dans le cytoplasme de s'accumuler dans la vacuole.

De même, ce mécanisme est également similaire à celui observé pour la détoxification de la cellule pour les métaux lourds ou le sel par exemple.

Compte tenu de ces résultats, les inventeurs ont élaboré une méthode pour identifier des protéines impliquées dans la reconnaissance et le transport ciblé de virus pathogènes dans les plantes, et cloner les gènes impliqués dans ces processus.

L'invention a donc pour but de fournir une méthode pour capturer les protéines cibles indispensables au cycle infectieux d'un virus pathogène notamment du virus RYMV et vise les protéines ainsi isolées.

Un procédé pour l'identification de marqueurs moléculaires du locus de résistance au RYMV est également décrit.

Sont visés également, en tant que tels, les fragments d'ADN tels que révélés par ce procédé, et utilisables en tant que marqueurs.

Sont visées en outre les applications de tels marqueurs, notamment pour définir d'autres marqueurs de haute spécificité vis-à-vis du locus de résistance et pour prédire un phénotype résistant.

Est visée tout particulièrement l'application desdits marqueurs pour établir la cartographie physique de la résistance et pour le clonage du gène.

Sont visés encore, en tant que produits, les séquences des amorces utilisées dans les techniques PCR mises en oeuvre.

La méthode d'isolement de protéines impliquées dans la reconnaissance et le transport ciblé d'un virus pathogène circulant via les plasmodermes dans une plante est caractérisée en ce qu'on soumet des échantillons renfermant des complexes desdites protéines avec des particules virales à une électrophorèse et un Western Blot en utilisant un anticorps monoclonal anti-protéine de capside, et on récupère les bandes non immunodétectées.

Selon une variante, le complexe est obtenu à partir de virus extrait de plantes sensibles infectées.

Le virus est plus particulièrement le virus de RYMV et on récupère des protéines de 5, 24, 42, 49, 59, 66, 70, 77 et 210 kDa.

Selon une autre variante, le complexe est obtenu à partir de virus purifié et mis en contact avec les protéines d'une suspension cellulaire de plante sensible.

En particulier, le virus est le virus de RYMV et on récupère des protéines de 24, 45, 51, 57, 63, 85 et au-delà de 120 kDa. L'application des protéines telles qu'obtenues par la méthode définie ci-dessus est visée en particulier pour le clonage des gènes de résistance à des virus pathogènes circulant via les plasmodermes dans une plante.

Est visé en outre l'identification de marqueurs du locus d'un gène majeur de résistance à RYMV, comprenant l'utilisation de marqueurs AFLP (Amplified Fragments Length Polymorphism) et faisant appel à la technique PCR.

Ce procédé d'identification est caractérisé en ce qu'il comprend

- l'amplification sélective de fragments d'ADN de riz d'une part d'individus résistants, d'autre part d'individus sensibles, descendant de variétés parentales, ces fragments ayant été préalablement soumis à une étape de digestion, puis de ligation pour fixer des adaptateurs complémentaires d'amorces ayant, à leur extrémité, un ou plusieurs nucléotides spécifiques, l'une des amorces du couple étant marquée aux fins de révélation,

- la séparation des produits d'amplification, par électrophorèse sur gel dans des conditions dénaturantes, et

- la comparaison des profils d'électrophorèse obtenus avec des mélanges de fragments issus de descendants résistants et des mélanges issus de descendants sensibles, avec les fragments provenant des variétés parentales, aux fins d'identification de bandes dont le polymorphisme est génétiquement lié au locus de résistance, cette identification étant suivie le cas échéant, à titre de validation, d'une vérification sur chacun des individus et du calcul du taux de recombinaison génétique entre le marqueur et le locus de résistance.

Dans un mode décrit, les fragments d'ADN sont obtenus par digestion des ADN génomiques de plantes résistantes d'une part, et de plantes sensibles d'autre part, et de leurs parents, à l'aide d'enzymes de restriction.

Des enzymes de restriction qui se sont révélées appropriées comprennent EcoRI et MseI.

De courtes séquences nucléotidiques sont fixées aux fragments de digestion (adaptateurs) pour générer des extrémités franches auxquelles sont ensuite fixés des adaptateurs.

Les amorces utilisées dans l'étape d'amplification sont complémentaires de ces adaptateurs avec, à leur extrémité 3', de 1 à 3 nucléotides qui peuvent être variables.

L'étape d'amplification est conduite avantageusement selon la technique PCR.

Des profils d'amplification spécifiques sont obtenus avec des couples d'amorces possédant à leur extrémité, respectivement, des motifs AAC et CAG, ACC et CAG, ou encore AGC et CAG.

Les séquences correspondant aux adaptateurs EcoRI et MseI sont respectivement GAC TGC GTA CCA ATT C (SEQ ID N°1) et GAT GAG TCC TGA GTA A (SEQ ID N° 2).

Les couples d'amorces mis en oeuvre pour l'amplification sont alors avantageusement choisis parmi E-AAC/M-CAG ; E-ACC/M-CAG ; et E-AGC/M-CAG ; dans lesquels E et M correspondent respectivement à SEQ ID N° 1 et SEQ ID N°2. D'autres couples sont donnés dans le tableau 6 dans les exemples.

L'étude comparative des profils d'amplification obtenus permet de révéler des bandes polymorphes spécifiquement présentes chez les variétés sensibles et leurs descendants sensibles, comme exposé dans les exemples, et correspondant en conséquence à des marqueurs de résistance.

En particulier, la révélation sur gel d'électrophorèse en conditions dénaturantes conduit à l'identification de 2 bandes marqueurs M1 et M2 respectivement de 510 pb et 140 pb.

Ces 2 bandes déterminent, d'après l'analyse des données de ségrégation, un segment chromosomique de 10 à 15 cM portant le locus de résistance et sont situées de part et d'autre de ce locus, à 5-10 cM.

Selon une disposition du procédé décrit, les bandes polymorphes identifiées en tant que marqueurs spécifiques du locus de la résistance au RYMV sont isolées à partir des gels. On opère avantageusement par excision des gels d'électrophorèse. Cette étape d'isolement est suivie d'une purification en procédant selon les techniques classiques. On dispose ainsi de fragments d'ADN.

Selon une autre disposition, lesdits fragments purifiés sont clonés dans un vecteur approprié, tel qu'un plasmide, introduit dans des cellules hôtes, notamment des cellules bactériennes comme celles de *E. coli.*

Selon encore une autre disposition, les fragments d'ADN purifiés et clonés sont séquencés.

Mettant à profit les séquences des inserts correspondant auxdits fragments d'ADN, est fourni également un procédé d'obtention de marqueurs de grande spécificité vis-à-vis du locus d'un gène majeur de résistance au RYMV. Ce procédé est caractérisé en ce qu'on définit des couples d'amorces PCR complémentaires de fragments de la séquence d'un insert donné, on procède à une amplification spécifique de l'insert à l'aide de ces couples d'amorces, puis on soumet les produits d'amplification à une migration sur gel d'électrophorèse.

Ces séquences d'ADN sont utilisables pour identifier un polymorphisme lié au locus de résistance dans une variété de riz à étudier suivant différents procédés, ainsi que décrit dans les exemples :

1) en identifiant directement un polymorphisme de taille de ces séquences d'ADN après amplification spécifique et séparation des fragments sur gel d'agarose,

2) en digérant les produits d'amplification par des enzymes de restriction pour séparer les produits de digestion sur gel d'agarose,

3) en utilisant ces séquences comme des sondes pour hybrider l'ADN de variétés de riz préalablement digérées par une enzyme de restriction et déterminer un polymorphisme de restriction.

Sont visées, en tant que produits, les bandes AFLP polymorphes telles qu'identifiées par le procédé défini ci-dessus, à partir d'ADN de plantes de riz, et le cas échéant isolées, purifiées et séquencées.

Ces bandes AFLP sont caractérisées en ce qu'elles sont spécifiquement mises en évidence dans une variété sensible au RYMV (IR64) et dans la fraction de plantes sensibles issues du croisement de cette variété avec la variété résistance Gigante comme décrit dans les exemples.

Sont visées tout spécialement les séquences d'ADN correspondant à ces bandes polymorphes, et qui permettent de définir un segment du chromosome 4 de 10-15 cM portant le locus de résistance au RYMV.

Compte tenu de leur procédé d'obtention, les bandes AFLP correspondent à des fragments de restriction et en particulier, conformément à un mode de réalisation du procédé décrit, à des fragments EcoRI - MseI.

Des fragments de ce type sont appelés marqueurs M1 et M2 et sont caractérisés par une taille, respectivement, de 510 pb et de 140 pb en gel d'électrophorèse en conditions dénaturantes.

Ces fragments sont caractérisés en ce qu'ils correspondent à des séquences d'ADN flanquant le locus de résistance et situés de part et d'autre de ce dernier à 5-10 cM.

Est décrit un ADNc tel que défini ci-dessus, caractérisé en ce que lesdites séquences d'ADN correspondant auxdites bandes polymorphes, portent le locus de résistance à RYMV et définissent un segment inférieur à 10cM.

Sont décrits également des fragments clonés dans des vecteurs tels que plasmides, ces vecteurs de clonage en tant que tels, caractérisés par le fait qu'ils comportent de tels fragments, et les cellules hôtes transformées à l'aide de ces vecteurs, tels que des cellules bactériennes comme *E. coli. e*st décrite notamment la séquence d'ADN correspondant au fragment identifié comme marqueur M1 et répondant à la séquence SEQ ID N° 3 suivante :

CGTGCTTGCTTATAGCACTACAGGAGA<u>AGGAAGGGGAACACAACAGC</u>
<u>C</u>ATGGCGAGCGAAGGTTCAACGTCGGAGAAACAGGCTGCGACGGGCA
GCAAGGTGCCGGCGGCGGATCGGAGGAAGGAAAAGGAGGAAATCGA
AGT<u>TTATGCTGGAGGGGCTTGACC</u>TAAGGGCAGATGAGGAGGAGGATG
TGGAATTGGAGGAAGATCTAGAGGAGCTTGAGGCAGATGCAAGATGG
CTAGCCCTAGCCACAGTTCATACGAAGCGATCGTTTAGTCAAGGGGCT
TTCTTTGGGAGT<u>ATGCGCTCAGCATGGAACTGC</u>GCGAAAGAAGTAGAT
TTCAGAGCAATGAAAGACAATCTGTTCTCGATCCAATTCAATTGTTTG
GGGGATTGGGAACGAGTTATGAATGAAGGTCCAT<u>GGACCTTTCGAGG</u>
<u>ATGTTC</u>GGTGCTCCTCGCAGAATATGATGGCTGGTCCAAGATTGAAT

La séquence d'ADN du marqueur M1 présente une taille de 471 pb.

Sont décrites encore, en tant que produits, les séquences de nucléotides utilisées comme amorces d'amplification en PCR.

De telles amorces comprennent les couples E-AAC/M-CAG ; E-ACC/M-CAG ; E-ACC/M-CAG ; dans lesquels E et M correspondent respectivement à SEQ ID N° 1 et SEQ ID N° 2.

D'autres amorces encore sont complémentaires de séquences identifiées dans la séquence du fragment désigné par marqueur M1. Il s'agit en particulier de séquences (5', 3') choisies parmi :

AGGAAGGGGAACACAACAGCC (21 pb) (SEQ ID N° 4)

TTATGCTGGAGGGGCTTGACC (21 pb) (SEQ ID N° 5)

GCAGTTCCATGCTGAGCGCAT (21 pb) (SEQ ID N° 6)

CCGAACATCCTCGAAAGGTCC (21 pb) (SEQ ID N° 7)

TCATATTCTGCGAGGAGCACC (21 pb) (SEQ ID N° 8)

Est décrite également la séquence d'ADN correspondant au fragment identifié comme marqueur M2 et répondant à la séquence SEQ ID N°9

<u>A</u><u>ATTCACCCC</u> <u>ATGCCCTAAG</u> TTAGGACGTT CTCAGCTTAG TGGTGTGGTA

GCTTTTTCTA TTTTCCTAAG CACCCATTGA AGTATTTTGC <u>ATTGGAGGTG</u>

<u>GCCTTAGGTT</u> <u>TGC</u>CTCTGTTA

La taille de M2 est de 120 pb.

Des amorces spécifiques complémentaires des séquences identifiées dans la séquence de M2 ont été définies. De telles séquences répondent aux enchaînements suivants (5', 3') :

SEQ ID N°10
AACCTAAGGCCACCTCCAAT
SEQ ID N°11
GCAAACCTAAGGCCACCTC
SEQ ID N°12
ATTCACCCCATGCCCTAAG

Selon encore un autre aspect, l'utilisation des séquences d'ADN obtenues avec les amorces ci-dessus pour définir des polymorphismes permettant l'identification de phénotypes résistants, est décrite.

Est décrit également un procédé d'identification de la séquence d'ADN portant le gène majeur de la résistance au RYMV. Ce procédé est caractérisé par le criblage d'une banque constituée de fragments d'ADN de 100 à 150 kb de la variété IR64 ou autre, telle que la banque BAC (Bacterial Artificial Chromosomes), clonés dans des bactéries, pour sélectionner le ou les clones de la banque renfermant les marqueurs définis ci-dessus et le gène de résistance au RYMV.

Une telle banque BAC est disponible auprès de l'IRRI.

Pour identifier le gène du ou des clones sélectionnés, on procède à l'identification à partir du jus brut de protéines extraites de plantes, de la fraction, puis de proche en proche, de la protéine qui mise en présence de virus purifié permet le mouvement de cellule à cellule dans la variété résistante. La ou les protéines candidates sont alors séquencées partiellement soit à partir de l'extrémité N-terminale, soit à partir de fragments internes libérés par hydrolyse. Des amorces peuvent être ainsi définies et sont utilisées pour amplifier des ADNc correspondants. Aux fins de validation, on vérifie que ces ADNc vont obligatoirement aller hybrider les clones BAC placés dans l'intervalle compris entre les marqueurs microsatellites.

En variante, on procède au sous-clonage du fragment BAC contenant le gène en éléments de plus petite taille sous forme de cosmides qui sont ensuite réordonnés de manière à couvrir l'ensemble du clone BAC de départ. Ces cosmides sont utilisés en transformation génétique pour réaliser un test de complémentation fonctionnelle permettant de valider la séquence contenue dans le cosmide et correspondant à l'ADNc isolé par l'approche protéine. Il s'agit en l'occurence de démontrer que la synthèse de la protéine responsable du mouvement du virus de cellule à cellule permet de rendre sensible la variété résistante.

Est décrit ainsi un ADNc capable de s'hybrider avec un clone BAC criblé à partir d'une banque constituée comme indiqué ci-dessus de fragments d'ADN de 100 à 150 kb d'une variété de riz telle que IR64, par exemple de banque BAC (Bacterial Artificial Chromosomes), ce clone BAC appartenant à un contig (ou ensemble de clones BAC chevauchant) de clones BAC renfermant les séquences d'ADN des marqueurs identifiés à partir de riz grâce au procédé défini ci-dessus.

Le gène de résistance peut être transféré à des variétés sensibles par voie conventionnelle grâce à l'utilisation de marqueurs génétiques spécifiques qui lui sont liés. Ainsi des variétés résistantes pourront être développées beaucoup plus facilement et beaucoup plus rapidement.

On notera de plus avec intérêt que la séquence de ce gène facilite l'accès à des gènes de résistance d'autres virus (Potyvirus par exemple) qui sont pathogènes d'autres plantes, mais caractérisés par le même mécanisme (mouvement de cellule à cellule). Sont fournis ainsi des moyens de grand intérêt pour l'amélioration des plantes basés sur des résistances naturelles aux pathogènes des végétaux.

D'autres caractéristiques et avantages seront donnés dans les exemples qui suivent, dans lesquels il est fait référence aux figures 1 à 15 qui représentent respectivement

- la figure 1 : le clonage du marqueur M1 dans le plasmide PGEMTeasy. La digestion du plasmide montre un fragment d'ADN de 510 pb correspondant à la bande M1 ;

- la figure 2 : l'amplification du marqueur M1 dans les quatre variétés de riz (Azucena, Gigante, IR64 et Tog5681) en utilisant les couples d'amorces
2-4) : 291 pb ; (2-5) : 310 pb ; (1-3) : 288 pb ;
(1-4) :406 pb ; (1-5) : 425 pb ; (2-3). Le fragment M1 est légèrement plus grand chez Tog5681 que chez les autres variétés ;

- la figure 3 : l'identification de sites de restriction sur la séquence du marqueur M1 chez les 4 variétés IR64, Azucena, Gigante et Tog5681 ;

- la figure 4 : la digestion du marqueur M1 avec l'enzyme HpaII après amplification PCR en utilisant les couples d'amorces (1-3), (1-4) et (1-5) sur les quatre variétés (Azucena, Gigante IR64 et Tog5681). La présence d'un site de restriction HpaII dans les variétés IR64 et Tog5681 libère un fragment de 86 pb qui réduit d'autant la taille du fragment amplifié,

- la figure 5 : la caractérisation du marqueur M1 sur les plantes sensibles et résistantes de la descendance F2 (IR64 x Gigante). Les plantes F2 résistantes ont le profil du parent résistant (IR64-absence du site HpaII), à l'exception d'un seul recombinant, les plantes résistantes ont le profil du parent sensible (IR64-présence du site HpaII) à l'exception de deux recombinants ;

- la figure 6 : la ségrégation du marqueur M1 dans la population HD (IR64 x Azucena) : IR64-Azucena-30 individus HD (IR64 x Azucena);

- la figure 7 : la carte de liaison génétique du chromosome 4 du riz avec le positionnement du marqueur M1 et l'identification de l'intervalle dans lequel se trouve le locus de résistance ;

- la figure 8 : l'hybridation du marqueur M1 utilisé comme sonde sur des membranes portant l'ADN des 4 variétés (IR64, Azucena, Gigante et Tog5681) digérées par 6 enzymes de restriction ApaI, KpnI, PstI, ScaI, HaeIII. La variété Tog5681 présente un profil de restriction différent des autres variétés pour l'enzyme ScaI qui peut être utilisée pour marquer le locus de résistance de cette variété ; et

- la figure 9 : l'hybridation du marqueur M1 utilisé comme sonde sur des membranes portant l'ADN d'individus issus de recroisement (IR64 x Tog5681) x Tog 5681 et digérés avec l'enzyme ScaI. Cette descendance est en ségrégation pour la résistance au RYMV. Les individus sensibles (5) montrent tous la bande d'IR64 associée à la bande de Tog5681 (individus hétérozygotes). Les individus résistants (9) ne montrent que la bande de Tog5681 à l'exception d'un individu recombinant,

- la figure 10 : la cartographie et l'ancrage du locus de résistance élevée au RYMV sur la carte IR64 x Azucena,

- la figure 11, le mouvement du virus de RYMV dans une plante, après inoculation dans une feuille,

- la figure 12, les chromatogrammes à partir de virus extraits de plantes sensibles infectées, et

- les figures 13 à 15, un chromatogramme après injection de virus, un gel SDS PAGE et un immunoblot avec un anticorps anti-protéine de capside.

Exemple 1 : Identification des variétés sources de résistance

Les variétés utilisées dans l'étude de la résistance et en particulier les deux variétés résistantes Gigante et Tog5681 ont été caractérisées grâce à des marqueurs microsatellites sur un échantillonnage représentatif de loci.

Le polymorphisme se manifeste par le nombre de répétitions d'un court motif nucléotidique, le plus souvent binucléotidique qui est caractéristique d'une variété donnée.

Sur un ensemble de loci, les allèles répertoriés permettent de disposer des caractéristiques spécifiques de chaque variété.

La mise en évidence de ces marqueurs microsatellites s'effectue par l'amplification de l'ADN avec des amorces spécifiques déterminées par Chen et *al.* (1), suivie d'une migration sur gel de polyacrylamide en conditions dénaturantes suivant le protocole décrit par les mêmes auteurs.

Le tableau 1 donne les résultats à partir d'un système de référence établi par Chen *et al.,* ci-dessus suivant lequel les allèles sont identifiés par le nombre de répétitions du motif comparativement à la variété IR36 qui sert de témoin. Les deux variétés Gigante et Tog5681 sont ainsi décrites spécifiquement sur 15 loci vis-à-vis de toutes autres variétés (les marqueurs microsatellites sont donnés dans la 1ère colonne).

**Tableau 1**

| Locus | Chr | Taille sur IR 36 | référence | IR36 | Gigante | IR64 | Azucena | TOG 568113 |
|-------|-----|------------------|-----------|------|---------|------|---------|------------|
| RM001 | 1 | 113 | (2) | n | n-26 | n | n-22 | n-26 |
| RM005 | 1 | 113 | (2) | n | n-6 | n-4 | n+16 | n-8 |
| RM011 | 7 | 140 | (2) | n | n-4 | n | n-24 | n-16 |
| RM018 | 7 | 157 | (2) | n | n+4 | n+6 | n+8 | n-6 |
| RM019 | 12 | 226 | (2) | n | n | n+21 | n-9 | n-21 |
| RM021 | 11 | 157 | (2) | n | n+8 | n | n-14 | n-32 |
| RM148 | 3 | 129 | (3) | n | n+6 | n | n | n+6 |
| RM167 | 11 | 128 | (3) | n | n+4 | n | n+32 | n+24 |
| RM168 | 3 | 116 | (3) | n | n-20 | n | n-20 | n-24 |
| RM232 | 3 | 158 | (1) | n | n-14 | n | n-12 | n-16 |

(suite)

| Locus | Chr | Taille sur IR 36 | référence | IR36 | Gigante | IR64 | Azucena | TOG 568113 |
|-------|-----|------------------|-----------|------|---------|------|---------|-----------|
| RM022 | 3 | 194 | (2) | n | n-2 | n | n-4 | n-2 |
| RM252 | 4 | 216 | (1) | n | n+38 | n+2 | n-20 | n+10 |
| RM255 | 4 | 144 | (1) | n | n | n | n | n |
| RM246 | 1 | 116 | (1) | n | n-12 | n-12 | n-16 | n-12 |
| RM231 | 3 | 182 | (1) | n | n+6 | n-22 | n-4 | n-12 |

Exemple 2 : Caractérisation de la résistance

La résistance a été caractérisée à partir de l'inoculation artificielle de jeunes plantules avec du virus comparativement à une variété témoin IR64 extrêmement sensible.

Le contenu en virus a été suivi pendant 60 jours après inoculation grâce à des tests ELISA sur les dernières feuilles émises.

Ces tests n'ont jamais pu mettre en évidence de signal significativement différent de plantes témoins non inoculées par le virus.

Une autre expérimentation a été réalisée en inoculant des protoplastes isolés des deux variétés Tog5681 et Gigante. Dans les deux cas, il possible de détecter la présence des protéines virales (protéine de la capside et protéine de mouvement P1), ainsi que l'accumulation d'ARN viral, qui témoignent de la capacité de ces protoplastes à multiplier le virus, et ceci de la même manière que les protoplastes de variétés sensibles comme IR64.

Ainsi, si on considère que la réplication, le mouvement de cellule à cellule, et le transport à longue distance à travers les vaisseaux, sont les trois étapes principales du déroulement du cycle infectieux dans la plante, la résistance de ces deux variétés réside le plus logiquement dans un blocage du virus au niveau des cellules infectées.

Exemple 3 : Génétique de la résistance

Différents croisements F1 ont été réalisés entre la variété d'*O. sativa* résistante (Gigante), une variété d'*O. glaberrima* résistante Tog5681 (également identifiée par l'ADRAO) et la variété de référence très sensible IR64 (sélectionnée à l'IRRI).

La culture du matériel végétal, les croisements et la production des descendances ont été réalisés dans les serres de l'IRD à Montpellier.

Les hybrides F1 obtenus entre les variétés sensibles et résistantes ont été testés pour la résistance au virus du RYMV par test ELISA et suivi des symptômes.

Ces hybrides F1 se sont tous révélés aussi sensibles que le parent sensible et ont donc montré que la nature de la résistance était récessive.

En revanche, les hybrides entre les deux sources de résistance Gigante et Tog5681 n'ont donné que des hybrides F1 résistants en faveur d'un seul et unique locus de résistance chez ces deux sources de résistance.

Ces résultats sont résumés dans le tableau 2 ci-après.

Ce tableau donne la distribution des réponses ELISA (A 405 nm) dans les feuilles infectées par voie systémique des hybrides F1, des backcross et des descendants F2 obtenus à partir des backcross entre la variété IR64 sensible et les 2 cultivars résistants Gigante et Tog5681.

TABLEAU 2

| descendances hybrides F1 | Présence de symptômes | Nombre de génotypes | Distribution des valeurs de DO | | | Moyenne Valeurs DO |
|--------------------------|-----------------------|---------------------|-------------------|-----------|-----|--------------------|
| | | | (0,01 - 0,05) | (0,9 -1) | >1 | |
| dérivés de Tog 5681 | | | | | | |
| F1 : (IR64 x Tog 5681) | Sensible | - | - | - | 10 | 1,9 |
| BCS: (IR64 x Tog 5681) x IR64 | Sensible | 19 | - | 4 | 15 | 1,6 |

(suite)

| descendances hybrides F1 | Présence de symptômes | Nombre de génotypes | Distribution des valeurs de DO | | | Moyenne Valeurs DO |
|---|---|---|---|---|---|---|
| | | | (0,01 - 0,05) | (0,9 -1) | >1 | |
| BCG : (IR64 x Tog 5681) x Tog 5601 | en ségrégation | 22 | 12 | - | 10 | - |
| Dérivés d'une plante fertile BCS | | | | | | |
| BCSF2 | sensible | 11 | - | - | 11 | 1,3 |
| BCS x IR64 | sensible | 1 | - | - | 1 | 1,9 |
| BCS x Tog5681 | sensible | 15 | - | - | 15 | 1,9 |
| dérivés de Gigante | | | | | | |
| F1 : (IR64 x Gigante) | | - | - | - | 10 | 1,9 |
| F2 : (IR64 x Gigante) | en ségrégation | 65 | 15 | - | 50 | - |
| F3 : (Gigante) x Tog5681) | sensible | - | 10 | - | - | 0,3 |

Les réponses ELISA sont obtenues à partir de :

i) 10 plantes régénérées par bouturage pour chaque combinaison hybride F1 ii) 1 plante régénérée pour chaque génotype interspécifique dérivé de backcross iii) tests directs sur des plantules jeunes (inoculation à 10 jours après germination et temps de lecture à 7 jours après inoculation) pour les descendances interspécifiques F2 et fertiles

En ce qui concerne Gigante, l'hérédité de la résistance a été confirmée par un test de résistance sur 55 familles F3 du croisement (IR64 x Gigante). Les résultats sont donnés dans le tableau 3.

Ce tableau donne la ségrégation de la résistance à RYMV dans les descendances F3 (IR64 x Gigante). L'inoculation est effectuée 10 à 17 jours après la germination avec l'isolat du Burkina Faso et les symtômes sont suivis 45 jours après l'inoculation.

Tableau 3

| Classes de résistance | Nombre de descendances | Nombre de plantes | | | Fréquence des plantes résistantes |
|---|---|---|---|---|---|
| | | Total | Sensible | Résistant | |
| Sensible | 15 | 191 | 191 | 0 | 0 |
| en ségrégation | 30 | 343 | 262 | 81 | 0,24 |
| | | | | | $x2 = 0,07$ (3:1) |
| Résistant | 4 | 45 | 14 | 31 | 0, 69 |
| très résistant | 6 | 87 | 0 | 87 | 1 |
| Résistant* | 7 | 73 | 23 | 50 | 0,68 |
| très résistant* | 4 | 56 | 0 | 56 | 1 |

* descendances F3 dérivées de plantes résistantes F2 analysées par tests ELISA

L'examen de ce tableau montre que :

- 1/4 de plantes F2 ne donne que des plantes résistantes dans les descendances F3, et sont homozygotes pour la résistance
- 1/4 de plantes F2 ne donne que des plantes sensibles dans les descendances F3, et sont homozygotes pour la sensibilité

- 1/2 des plantes F2 sont en ségrégation pour la résistance et donnent des plantes sensibles et résistantes avec la même proportion (3:1) dans les descendances F3.

L'ensemble des résultats s'accorde parfaitement avec un seul gène de résistance récessif présent chez les deux variétés Gigante et Tog5681.

Exemple 4 : Identification des marqueurs de résistance M1 et M2 selon le protocole AFLP

a - Obtention de pools d'ADN

Les feuilles de 10 plantes sensibles et de 10 plantes résistantes issues d'une F2 (IR64 x Gigante) ont été prélevées pour extraire leur ADN.

Les ADN ont été ensuite mélangés de manière stoechiométrique pour constituer deux pools d'ADN correspondant respectivement à 10 plantes F2 sensibles ou résistantes avec une concentration finale du mélange de 50 ng/$\mu$l. Ces mélanges ont servi de base à l'identification de marqueurs de résistance par la méthode AFLP (Amplified Fragments Length Polymorphism) pour polymorphisme de longueur de fragments amplifiés qui a été développée par Zabeau et *al* (4), et Vos et *al.*(5). Les produits utilisés se présentent sous forme d'un kit commercial (Gibco BRL) délivré par Keygene & Life Technologies.

b - Obtention de fragments de restriction

250 ng de chacun des pools d'ADN à 50 ng/$\mu$l et des parents sont digérés simultanément par deux enzymes de restriction (EcoRI et MseI).

Réaction digestion (25 $\mu$l) :

5 $\mu$l d'ADN (50 ng/ml)
0,2 $\mu$l (2 U) de EcoRI (10 U/$\mu$l)
0,2 $\mu$l (2 U) de MseI (5 U/$\mu$l)
5 $\mu$l de tampon T4 ligase 5X
14,5 $\mu$l de $H_2O$.

La réaction de digestion s'effectue pendant deux heures à 37°C, puis 15 min. à 70°C pour inactiver les enzymes de restriction. Après digestion, il est procédé à une réaction de ligation.

Réaction de ligation (50 $\mu$l) :

25 $\mu$l du milieu réactionnel de double digestion
1 $\mu$l d'adaptateur EcoRI
1 $\mu$l d'adaptateur MseI
5 $\mu$l de tampon T4 Ligase 5X
1 $\mu$l (1 U) de ligase (10 U/$\mu$l)
17 $\mu$l $H_2O$.

La réaction de ligation s'effectue à 37°C, pendant 3 heures, suivie d'une inactivation de l'enzyme à 60°C pendant 10 min.

c - Amplification

L'amplification proprement dite a été réalisée en deux étapes : préamplification et amplification spécifique.

C1 - Réaction de pré amplification (50 $\mu$l)

5 $\mu$l du milieu réactionnel renfermant l'ADN digéré et fixé aux adaptateurs, dilué au 1/10

0,5 $\mu$l d'amorce EcoRI (150-ng/$\mu$l)

0,5 $\mu$l d'amorce MseI (150 ng/$\mu$l)

2 $\mu$l de mélange de nucléotides 5 mM

5 $\mu$l de tampon 10 X, Promega

5 $\mu$l de MgCl$_2$ 25 mM

0,2 $\mu$l (1 U) de Taq polymérase (5 U/$\mu$l)

31,8 $\mu$l de H$_2$O.

Les caractéristiques de la pré-amplification par PCR sont les suivantes :

20 cycles avec    dénaturation : 30 sec à 94°C
hybridation : 30 sec à 56°C
élongation : 1 min à 72°C

L'amplification sélective se fait à partir d'un aliquote de la première amplification diluée au 1/30 en utilisant des amorces ayant 3 nucléotides sélectifs à l'extrémité 3', et en marquant l'une des amorces pour révéler les bandes sur un film autoradiographique.

On utilise les couples d'amorces suivants :

E-AAC/M-CAG
E-ACC/M-CAG
E-AGC/M-CAG,
dans lesquels
E répond à la séquence
GAC TGC GTA CCA ATT C (SEQ ID N°1), et
M à la séquence
GAT GAG TCC TGA GTA A (SEQ ID N° 2).

La température d'hybridation est diminuée de 0,7°C par cycle, pendant les 11 cycles suivants :

20 derniers cycles    dénaturation : 30 sec à 90°C
hybridation : 30 sec à 56°C
élongation : 1 min à 72°C

On procède au marquage de l'amorce EcoRI (ramené à un tube de 0,5 $\mu$l):

0,18 $\mu$l de l'amorce EcoRI (5ng)

0,1 $\mu$l de $\gamma^{33}$P ATP (10 mCu/$\mu$l)

0,05 $\mu$l de tampon kinase 10 X

0,02 $\mu$l (0,2U) de T4 polymérase kinase (10U/$\mu$l)

0,15 $\mu$l de H$_2$O.

La réaction de marquage se fait à 37°C pendant 1 heure et est arrêtée par 10 minutes à 70°C.

C2 - Réaction d'amplification spécifique

20 $\mu$l :

0,5 $\mu$l d'amorce EcoRI marquée

5 $\mu$l du milieu réactionnel de pré-amplification, dilué au 1/30,

0,3 $\mu$l d'amorce MseI (100 ng/$\mu$l)

0,8 $\mu$l de mélange de nucléotides 5 mM

2 $\mu$l de tampon 10 X Promega

2 $\mu$l de MgCl$_2$ 25 mM

0,1 $\mu$l (0,5 U) de Taq polymérase (5 U/$\mu$l)

9,3 $\mu$l de $H_2O$.

Les caractéristiques de l'amplification sont les suivantes :

32 cycles avec

pour le premier cycle :

dénaturation : 30 sec à 94°C
hybridation : 30 sec à 65°C
élongation : 1 min à 72°C

les 11 cycles suivants : les mêmes conditions que précédemment, avec diminution à chaque cycle de 0,7°C de la température d'hybridation ; et pour
les 20 derniers cycles :

dénaturation : 30 sec à 90°C
hybridation : 30 sec à 56°C
élongation : 1 min à 72°C

d - Electrophorèse et Autoradiographie

A la fin de la réaction d'amplification, 20 $\mu$l de tampon de charge sont ajoutés (98 % de formamide, 0,005 % de xylène cyanol et 0,005 % de bleu de bromophénol). Les produits d'amplification sont séparés par électrophorèse sur gel de polyacrylamide dénaturant (6 % d'acrylamide, 8 M d'urée) avec un tampon de migration TBE (Tris 18 mM, EDTA 0,4 mM, acide borique 18 mM, pH 8,0) pendant 3 heures de migration à puissance de 50 watts. Après migration, le gel est fixé dans une solution 1V d'acide acétique/2V d'éthanol absolu pendant 20 minutes. Le gel est transféré sur un papier Wattman 3M et séché pendant 45 minutes à 80°C avec un sécheur de gel. Le gel est placé dans une cassette avec un film ultrasensible. L'autoradiographie est révélée après deux jours d'exposition. La comparaison des profils obtenus chez les parents et les pools de plantes sensibles ou résistantes permet d'identifier des bandes présentes dans l'un des pools, mais absentes dans l'autre. Ces bandes candidates au marquage de la résistance sont ensuite vérifiées individuellement sur chacune des plantes composant les pools d'ADN.

e - Résultats

L'étude des résultats obtenus montre que les deux marqueurs appelés M1 et M2 sont présents chez le parent sensible (IR64) ainsi que dans toutes les plantes F2 (IR64 x Gigante) composant le pool de plantes sensibles, alors que cette bande est absente chez le parent résistant (Gigante) et qu'un seul individu du pool résistant manifeste cette bande. Le même type de variation est observé dans le recroisement (IR64 x Tog5681) x Tog5681. Les autres marqueurs identifiés dans cette analyse (M3 à M6) montrent aussi la même variation:

- présence des bandes chez le parent sensible et le pool des plantes sensibles F2 (IR64 x Gigante) ainsi que les plantes sensibles du recroisement (IR64 x Tog5681) x Tog5681.
- absence de bande chez les parents résistants Gigante et Tog5681, chez le pool des plantes résistantes F2 (IR64 x Gigante) et chez les plantes résistantes du recroisement (IR64 x Tog5681) x Tog5681.

Les données de ségrégation entre les marqueurs AFLP M1 à M6 et le locus de résistance pour les pools F2 (IR64 x Gigante) et le backcross interspécifique (IR64 x Tog5681) x Tog5681 sont résumées dans les tableaux 4 et 5. L'analyse des données de ségrégation et des rares recombinants observés dans les deux croisements permet d'évaluer les taux de recombinaison entre ces différents marqueurs et le locus de résistance. En particulier, les marqueurs M1 d'une part et les marqueurs M2 à M6 d'autre part déterminent un segment inférieur à 10-15 cM portant le locus de résistance. M1 et M2 sont ainsi à moins de 5-10 cM et placés de part et d'autre de ce locus.

### TABLEAU 4

| Résistance/Marqueur M1 | Nombre d'individus observés | | | | | | |
|---|---|---|---|---|---|---|---|
| Phénotype | Résistant | | | | Sensible | | |
| Génotype résistance RYMV | tt/gg | tt | gg | lt lt | lt | lt | |
| Marqueur AFLP | -/- | +/- | +/ | -/- +/ | + | -/- | |
| Pool F2 résistant (IR64 x gigante) | 10 | - | 1 | - | - | - | - |
| Pool F2 sensible (IR64 x gigante) | - | - | - | - | - | 0 | |
|  |  |  |  | 10 |  |  | |
| Backcross interspécifique Tog5681 | 11 | 1 | - | 0 | 8 | - | - |

| Résistance/Marqueur M2,M3,M4,M6 | Nombres d'individus observés | | | | | | |
|---|---|---|---|---|---|---|---|
| Phénotype | Résistant | | | | Sensible | | |
| Génotype résistance RYMV Marqueur AFLP | tt/gg -/- | tt +/- | gg +/ | lt -/- | ll +/- | | ll -/- | ll +/ |
| Pool F2 résistant (IR64 x gigante) | 11 | - | 0 | - | - | - | - | - |
| Pool F2 sensible (IR64 x gigante) | - | - | - | - | - | | 0 | 10 |
| Backcross interspécifique Tog5681 | 10 | 2 | - | 0 | 8 | | - | - |

| Résistance/Marqueur M5 | Nombre d'individus observés | | | | | |
|---|---|---|---|---|---|---|
| Phénotype | Résistant | | | | Sensible | |
| Génotype résistace RYMV | tt/gg | tt | gg | lt ll | lt | ll |
| Marqueur AFLP | -/ | +/- | +/ | -/- +/ | +/- | -/- |
| Pool F2 résistant (IR64 x gigante) | 11 | - |  | - | - | - |
|  | 0 |  |  | - |  |  |
| Pool F2 sensible (IR64 x gigante) | - | - |  | - | - | 0 |
|  | - |  |  | 10 |  |  |
| Backcross interspécifique Tog5681 | 9 | 3 |  | 0 | 8 | - |
|  | - |  |  | - |  |  |

### TABLEAU 5

| Marqueur M1/Marqueurs M2,M3,M4,M6 | Nombre d'individus observés | | | |
|---|---|---|---|---|
| Génotype M1 | -/* | +/* | -/- | -/- |
| Génotype M2,M3,M4,M6 | +/* | -/- | +/* | -/- |
| Pool F2 résistant (IR64 x gigante) | 0 | 1 | 0 | 10 |
| Pool F2 sensible (IR64 x gigante) | 10 | 0 | 0 | 0 |
| Backcross interspécifique Tog5681 | 11 | 2 | 2 | 11 |

| Marqueur M1/Marqueur M5 | Nombres d'individus observés | | | |
|---|---|---|---|---|
| Génotype M1 | -/ * | +/* | -/- | -/- |
| Génotype M5 | +/* | -/- | +/* | -/- |
| Pool F2 résistant (IR64 x gigante) | 0 | 1 | 0 | 10 |
| Pool F2 sensible (IR64 x gigante) | 10 | 0 | 0 | 0 |
| Backcross interspécifique Tog5681 | 11 | 2 | 3 | 10 |

(suite)

| Marqueur M1/Marqueur M5 | Nombres d'individus observés | | | |
|---|---|---|---|---|

| Marqueur M5/Marqueurs M2,M3,M4,M6 | Nombre d'individus observés | | | |
|---|---|---|---|---|
| Génotype M5 | +/* | +/* | -/- | -/- |
| Génotype M2,M3,M4,M6 | +/* | -/- | +/* | -/- |
| Pool F2 résistant (IR64 x gigante) | 0 | 0 | 0 | 11 |
| Pool F2 sensible (IR64 x gigante) | 10 | 0 | 0 | 0 |
| Backcross interspécifique Tog5681 | 13 | 1 | 0 | 12 |
| * : (-) backcross interspécifique Tog5681.(+ ou-) pool F2 | | | | |

Exemple 5 : Isolement du marqeur M1

Une nouvelle amplification, avec le même couple d'amorces, a été réalisée, suivie d'une migration sur gel de polyacrylamide dans les mêmes conditions que celles énoncées ci-dessus. La révélation a été faite par une coloration au nitrate d'argent, avec le kit silver staining (Promega), pour visualiser directement les bandes sur le gel. Après révélation, la bande M1 a été excisée du gel, puis l'ADN a été élué dans 50 $\mu$l d'eau à 4°C pendant une nuit.

Un aliquot de 5 $\mu$l a été prélevé et réamplifié avec les mêmes couples d'amorces avec un marquage au P[33]. Le produit d'amplification a été séparé à nouveau sur gel d'acrylamide dénaturant à 6%, et comparé aux parents et aux pools sensibles et résistants. La piste correspondant à ce produit d'amplification montre une seule bande de 510 pb migrant exactement au même niveau que la bande d'origine qui avait été excisée. Un autre aliquot de 5 $\mu$l a été également amplifié avec les mêmes amorces et a été séparé sur gel d'agarose à 1,8%. La bande correspondant à la taille attendue (510pb) a été à nouveau excisée et purifiée avec un kit gene clean (Promega).

Exemple 6 : Clonage et Séquençage du Marqueur M1

. clonage

3 $\mu$l du produit de purification ont été utilisés pour une réaction de clonage pendant une nuit à 37°C.

3 $\mu$l de produit de purification
1 $\mu$l de vecteur PGEMTeasy
1 $\mu$l de T4 Tampon ligase 10 X
1 $\mu$l de T4 DNA Ligase
4 $\mu$l de H$_2$O

La transformation a été réalisée avec la souche *E. coli* JM109 en ajoutant 5 $\mu$l du produit de clonage à 100 $\mu$l de cellules compétentes de *E. Coli* JM109. Une préculture a été réalisée sur milieu de culture LB pendant 1 heure, à 37 °C. Ensuite les bactéries ont été étalées sur boîte de Pétri contenant de l'agar à 1/1000 d'ampicilline. 50 $\mu$l d'IPTG-XGal sont ajoutés juste avant l'étalement des bactéries pour sélectionner les bactéries transformées. Une colonie blanche (transformée) a été sélectionnée et remise en culture dans les mêmes conditions (Agar plus ampicilline).

A partir de cette culture, une mini-préparation d'ADN plasmidique a été réalisée avec le kit Wizard plus (Promega). L'ADN plasmidique contenant l'insert a été digéré avec l'enzyme EcoRI pour vérifier la présence du marqueur M1. Un gel d'agarose à 1,8% a permis de vérifier la présence de la bande de 3 kb correspondant au plasmide et de la bande de 510 pb corespondant au marqueur M1 (photo 1).

. Séquençage

La séquence de l'insert (SEQ ID N° 3) est la suivante (5', 3') :

## SEQ ID N° 3

```
      20         30         40         50         60         70
GTGCTTGCTTATAGCACTACAGGAGAAGGAAGGGGAACACAACAGCC
ATGGCGAGCGAAGGTTCAACGTCGGAGAAACAGGCTGCGACGGGCAG
CAAGGTGCCGGCGGCGGATCGGAGGAAGGAAAAGGAGGAAATCGAA
GTTATGCTGGAGGGGCTTGACCTAAGGGCAGATGAGGAGGAGGATGT
GGAATTGGAGGAAGATCTAGAGGAGCTTGAGGCAGATGCAAGATGGC
TAGCCCTAGCCACAGTTCATACGAAGCGATCGTTAGTCAAGGGGCTT
TCTTTGGGAGTATGCGCTCAGCATGGAACTGCGCGAAAGAAGTAGATT
TCAGAGCAATGAAAGACAATCTGTTCTCGATCCAATTCAATTGTTTGG
GGGATTGGGAACGAGTTATGAATGAAGGTCCATGGACCTTTCGAGGAT
GTTCGGTGCTCCTCGCAGAATATGATGGCTGGTCCAAGATTGAAT
```

Les séquences correspondant aux amorces utilisées pour les amplifications AFLP ont été retrouvées et montrent que la bande correspond à un fragment de restriction (EcoRI - MseI).

En déduisant les séquences correspondant aux amorces, la taille réelle du fragment d'ADN de riz cloné est de 471 pb.

L'utilisation des différents couples d'amorces (1-3), (1-4), (1-5) d'une part et (2-3), (2-4), (2-5) d'autre part permet de valider le clonage de la bande AFLP M1. L'amplification de l'ADN des variétés utilisées dans les croisements avec ces amorces ne montre qu'une seule bande. Le fragment correspondant à la variété Tog56581 est un peu plus important que celui des autres variétés (fig. 2).

Exemple 7 : Transformation de la séquence M1 en marqueur polymorphe

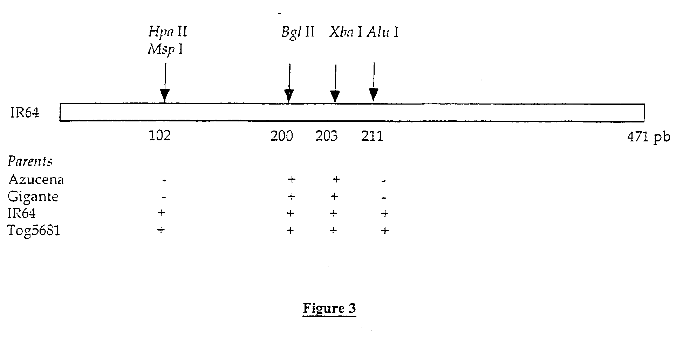

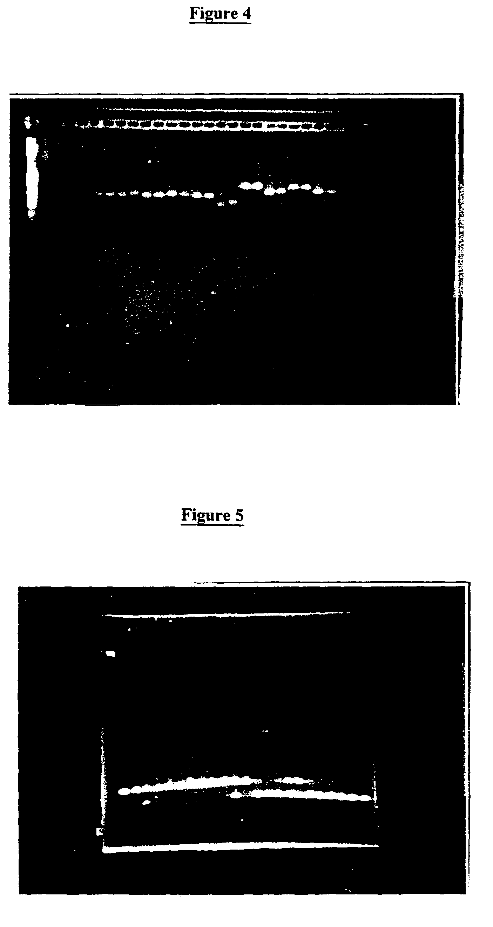

Un polymorphisme pour le marqueur M1 a été déterminé entre les parents de la population haploïde doublée (IR64 x Azucena). Cette population compte plus de 300 marqueurs répartis sur les 12 chromosomes du riz. Pour cela, on s'est appuyé sur les sites de restriction de la séquence du marqueur M1 déterminée sur le parent IR64 (fig. 3). Les amorces (1-3), (1-4) et (1-5) ont été utilisés pour amplifier l'ADN des parents des croisements qui a ensuite été digéré par des enzymes de restriction. Le site de restriction HpaII/MspI libère un fragment de 86 pb lorsque l'amorce 1 est utilisée. Ce site est absent chez les variétés Gigante et Azucena. (fig. 4).

Le marqueur a été testé sur les individus F2 du pool sensible et du pool résistant du croisement (IR64 x Gigante). Tous les individus résistants ont le profil de la variété Gigante (absence du marqueur AFLP M1 associée à l'absence du site de restriction HpaII/MspI) à l'exception de l'individu (5.11). Les individus sensibles présentent le site de restriction HpaII/MspI à l'état homozygote comme la variété IR64 à l'exception de deux individus hétérozygotes qui sont recombinés (fig. 5).

La séquence du marqueur M1 que l'on peut amplifier par des amorces spécifiques correspond bien au marqueur AFLP M1. La digestion par l'enzyme HpaII/MspI permet de distinguer l'allèle venant du parent sensible (IR64) du parent résistant (Gigante).

Ces nouvelles données permettent de conforter la position du locus de résistance entre les marqueurs M1 et M2 et d'estimer les taux de recombinaison à 0,065 $\pm$ 0,045 pour la distance entre M1 et le locus de résistance et 0,11 $\pm$ 0,047 pour la distance entre les marqueurs M1 et M2.

Exemple 8 : Cartographie du marqueur M1

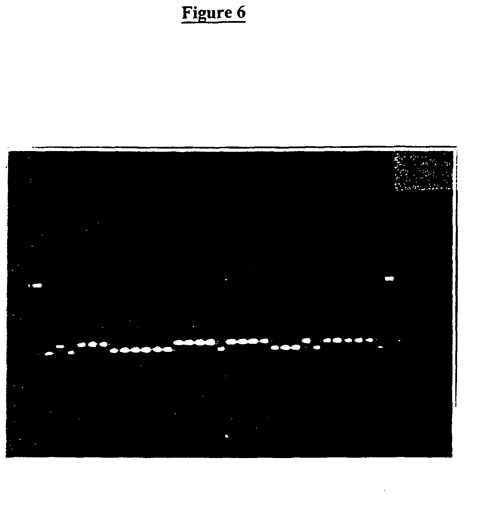

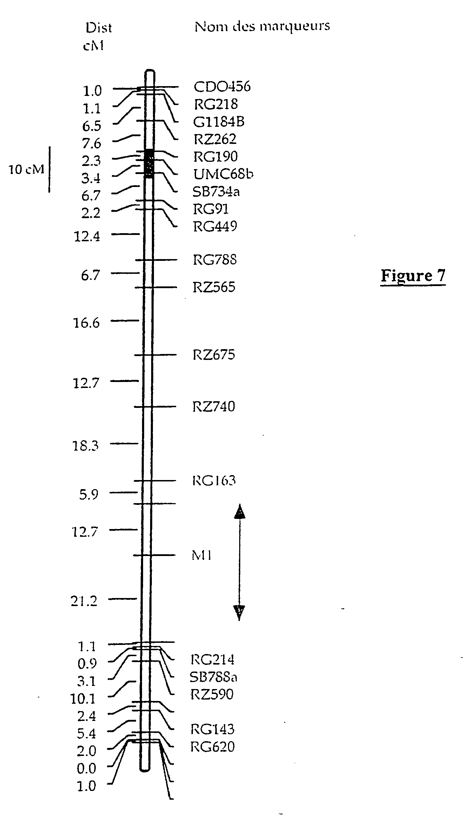

Soixante individus de la population (IR 64 x Azucena) ont été passés pour le marqueur M1 : amplification avec les amorces (1-3) et digestion par l'enzyme HpaII/MspI, suivie d'une séparation des fragments sur un gel d'agarose à 2,5 %. La ségrégation du marqueur M1 est sans distorsion (fig. 6). Les résultats permettent de cartographier le marqueur M1 en utilisant un logiciel de cartographie (Mapmaker V3), qui permet de placer le marqueur M1 sur le chromosome 4 entre les marqueurs RG 163 et RG 214 (fig. 7). Cet intervalle représente la zone où se situe le locus de résistance au RYMV.

La cartographie du gène de résistance au RYMV sur le chromosome 4 de la carte génétique du riz permet d'identifier des marqueurs plus proches du locus de résistance. Il s'agit en particulier de marqueurs microsatellites RM252 et RM273 ou tout autre marqueur à l'intérieur de l'intervalle (4-5cM) défini par ces marqueurs permettant d'identifier un polymorphisme entre les parents IR64 et Gigante tel que les marqueurs RFLP issus de banques génomiques ou d'ADNc, les microsatellites, les marqueurs AFLP ou les marqueurs dérivés de la cartographie physique de la région comme les clones BAC, YAC ou leurs cosmides.

Les marqueurs identifiés conformément à l'invention ou tout autre marqueur situé dans cet intervalle permettant d'identifier un polymorphisme entre des variétés résistantes tel que Gigante ou *O. glaberrima* avec des variétés de riz sensibles au RYMV peuvent être utilisés pour le transfert de la résistance au RYMV dans des variétés sensibles par recroisement successifs suivis de sélection assistée par marqueurs.

Exemple 9 : Marquage du locus de résistance de la variété Tog5681

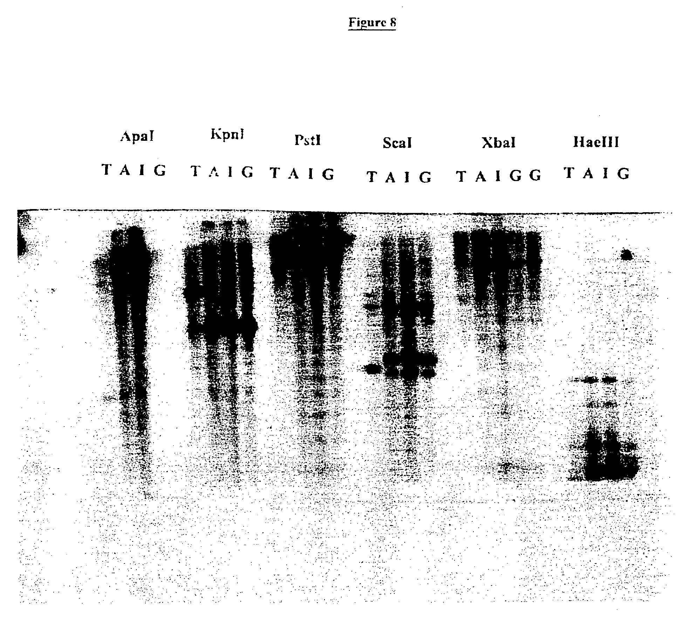

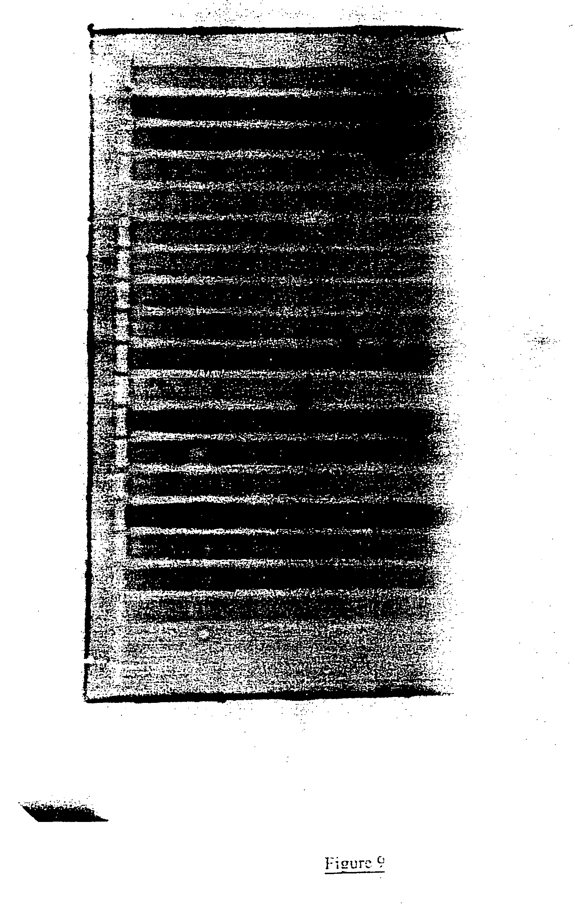

La présence du site de restriction HpaII/MspI dans la variété Tog5681 ne permet pas d'utiliser la stratégie de l'exemple 8 pour vérifier que le marqueur M1 est aussi un marqueur de la résistance provenant de Tog5681. Aussi, les 4 variétés Azucena, Gigante, IR64 et Tog5681 ont été digérées avec 12 enzymes de restriction (BamHI, Bg/II, DraI, EcoRI, EcoRV, HindIII, ApaI, KpnI, PstI, ScaI, XbaI, HaeIII) pour identifier un polymorphisme de restriction en utilisant la séquence d'ADN du marqueur M1 comme sonde. L'enzyme ScaI permet d'identifier un polymorphisme entre IR64 et Tog5681 (fig. 8). Ce polymorphisme a été utilisé pour valider le marqueur M1 sur un recroisement (IR64 x Tog5681) x IR64 en ségrégation pour la résistance. 5 individus sensibles de ce recroisement ont été testés et montrent tous la bande caractéristique d'IR64. Les 9 individus résistants ne montrent que la bande de Tog5681 à l'exception d'un seul qui est recombinant (fig. 9). Le polymorphisme de restriction mis en évidence par l'enzyme ScaI en utilisant le marqueur M1 comme sonde est donc bien lié au locus de résistance de Tog5681. L'analyse génétique et l'idendification de marqueurs de résistance sont cohérentes pour considérer que le marqueur M1 cartographie bien le même locus de résistance chez les deux variétés Gigante et Tog5681.

**Exemple 10**: Clonage et séquençage du marqueur M2 en marqueur PCR-spécifique

La bande AFLP obtenue avec le couple d'amorces E-ACC/M-CAG et correspondant à la bande M2 visible chez le parent sensible (IR64) et présente chez tous les individus composant le pool sensible a été clonée suivant le même protocole que pour le marqueur M1. La séquence correspondant à cette bande a été déterminée et 3 amorces ont été définies (1 forward - 2 reverse) pour permettre la transformation de ce marqueur en marqueur PCR-spécifique. Séquence du marqueur M2 (120pb) (SEQ ID N°9) :

AATTCACCCC ATGCCCTAAG TTAGGACGTT CTCAGCTTAG

TGGTGTGGTA GCTTTTTCTA TTTTCCTAAG CACCCATTGA

AGTATTTTGC ATTGGAGGTG GCCTTAGGTT TGCCTCTGTTA

Amorces :

(SEQ ID N°10) : AACCTAAGGCCACCTCCAAT (droite)
(SEQ ID N°11) : GCAAACCTAAGGCCACCTC (droite)
(SEQ ID N°12) : ATTCACCCCATGCCCTAAG (gauche)

Les conditions suivantes sont utilisées pour amplifier simultanément les marqueurs M1 et M2

- tampon 10X Proméga 1,5 $\mu$l
- MgCl$_2$ Proméga 1,5 $\mu$l
- dNTP (5 mM) 0,6 $\mu$l
- amorce M1-1 (10 $\mu$M) 0,15 $\mu$l
- amorce M1-4 (10 mM) 0,15 $\mu$l
- amorce M2-1 (10 mM) 0,15 $\mu$l
- amorce M2-2 (10 mM) 0,15 $\mu$l
- H$_2$O 7,74 $\mu$l

- Taq Polymerase 0,06 $\mu$l
- ADN (5 ng/$\mu$l) 3$\mu$l

Programme PCR

- 5mn à 94°C

- 1mn à 94°C

- 30 s à 59°C

- 1 mn à 72°C

- 35 cycles

- 5 mn à 72°C

- 10 mn à 4°C

Le marqueur M2 peut être amplifié seul avec une température d'hybridation de 60.5°C, les autres paramètres restant inchangés. Dans ces conditions d'amplification, le marqueur M2 apparaît comme un marqueur dominant se caractérisant par une présence de bande chez le parent sensible (IR64) et absence de bande chez le parent (Gigante).
Exemple 11 : Création d'une population de plantes résistantes recombinantes entre les marqueurs M1 et M2 pour ordonner à l'intérieur de cet intervalle les marqueurs AFLP candidats au marquage de la résistance 750 individus F2 (IR64 x Gigante) ont été inoculés artificiellement avec le virus RYMV (souche BF1). Les plantes sans symptômes ont été repiquées en serre soit 188 individus. Par la suite, des tests complémentaires basés sur des tests Elisa et des tests de descendances ont permis d'éliminer une dernière fraction de 50 plantes sensibles. Les 138 plantes restantes et homozygotes pour la résistance ont été systématiquement génotypées pour les deux marqueurs M1 et M2 comme précédemment définis. 45 individus ont été ainsi sélectionnés (38 recombinants par rapport à M1 ; 7 recombinants par rapport à M2) et 2 doubles recombinants. Ces individus recombinants ont servi à l'ordonnancement des marqueurs AFLP dans l'intervalle entre M1 et M2.
Ces résultats sont résumés dans le tableau 6 suivant :

### TABLEAU 6

| Sélection d'une sous-population F2 (IR64 x Gigante) recombinante dans l'intervalle des marqueurs M1-M2 | | |
|---|---|---|
| Etapes réalisées F2 (IR64 x Gigante) | Nombre de plantes | % |
| Inoculation des plantes F2 (10 jours après semis) | 768 | |
| Transplantation en serre (5 semaines après inoculation) | 188 | |
| Elimination de plantes sensibles | 50 | |
| (suivi des symptômes - Test Elisa et Test de descendance) | | |
| Sélection de plantes résistantes homozygotes pour le gène de résistance élevée | 138 | 17,9 |
| Génotypage des individus sélectionnés pour les marqueurs M1 et M2 | | |
| Plantes recombinées par rapport à M1 | 36 | 18,8 |
| Plantes recombinées par rapport à M1 et M2 | 2 | 1,4 |
| Plantes recombinées par rapport à M2 | 7 | 5,1 |

Exemple 12 : Criblage de marqueurs AFLP pour sélectionner de nouveaux marqueurs candidats à la résistance

Un total de 328 couples d'amorces EcoRI/MseI, chacun défini par 3 nucléotides, a été utilisé suivant le protocole précédemment défini. Ces amorces sont données dans le tableau 7 ci-après.

## TABLEAU 7

| N° de combinaison | Amorce EcoRI | Amorce MseI | N° de combinaison | Amorce EcoRI | Amorce MseI | N° de combinaison | Amorce EcoRI | Amorce MseI |
|---|---|---|---|---|---|---|---|---|
| 1 | AAC | CAA | 55 | ACA | CTG | 109 | ACG | AGG |
| 2 | AAC | CAC | 56 | ACA | CTT | 110 | ACG | AGT |
| 3 * | AAC | CAG | 57 | ACA | AAC | 111 | ACT | CAA |
| 4 | AAC | CAT | 58 | ACA | AAG | 112 | ACT | CAC |
| 5 | AAC | CCA | 59 | ACA | AAT | 113 | ACT | CAG |
| 6 | AAC | CCT | 60 | ACA | ACA | 114 | ACT | CAT |
| 7 | AAC | CGA | 61 | ACA | ACC | 115 | ACT | CCA |
| 8 | AAC | CGT | 62 | ACA | ACG | 116 | ACT | CCT |
| 9 | AAC | CTA | 63 | ACA | ACT | 117 | ACT | CGA |
| 10 | AAC | CTC | 64 | ACA | AGC | 118 | ACT | CGT |
| 11 | AAC | CTG | 65 | ACA | AGG | 119 | ACT | CTA |
| 12 | AAC | CTT | 66 | ACA | AGT | 120 | ACT | CTC |
| 13 | AAC | AAC | 67 | ACC | CAA | 121 | ACT | CTG |
| 14 | AAC | AAG | 68 | ACC | CAC | 122 | ACT | CTT |
| 15 | AAC | AAT | 69 * | ACC | CAG | 123 | ACT | AAC |
| 16 | AAC | ACA | 70 | ACC | CAT | 124 | ACT | AAG |
| 17 | AAC | ACC | 71 | ACC | CCA | 125 | ACT | AAT |
| 18 | AAC | ACG | 72 | ACC | CCT | 126 | ACT | ACA |
| 19 | AAC | ACT | 73 | ACC | CGA | 127 | ACT | ACC |
| 20 | AAC | AGC | 74 | ACC | CGT | 128 | ACT | ACG |
| 21 | AAC | AGG | 75 | ACC | CTA | 129 | ACT | ACT |
| 22 | AAC | AGT | 76 | ACC | CTC | 130 | ACT | AGC |
| 23 | AAG | CAA | 77 | ACC | CTG | 131 | ACT | AGG |
| 24 | AAG | CAC | 78 | ACC | CTT | 132 | ACT | AGT |
| 25 | AAG | CAG | 79 | ACC | AAC | 133 | AGA | CAA |
| 26 | AAG | CAT | 80 | ACC | AAG | 134 | AGA | CAC |
| 27 | AAG | CCA | 81 ** | ACC | AAT | 135 | AGA | CAG |
| 28 | AAG | CCT | 82 | ACC | ACA | 136 | AGA | CAT |
| 29 | AAG | CGA | 83 | ACC | ACC | 137 | AGA | CCA |
| 30 | AAG | CGT | 84 | ACC | ACG | 138 | AGA | CCT |
| 31 | AAG | CTA | 85 | ACC | ACT | 139 | AGA | CGA |
| 32 | AAG | CTC | 86 | ACC | AGC | 140 | AGA | CGT |
| 33 | AAG | CTG | 87 | ACC | AGG | 141 | AGA | CTA |
| 34 | AAG | CTT | 88 | ACC | AGT | 142 | AGA | CTC |
| 35 | AAG | AAC | 89 | ACG | CAA | 143 | AGA | CTG |
| 36 | AAG | AAG | 90 | ACG | CAC | 144 | AGA | CTT |
| 37 | AAG | AAT | 91 ** | ACG | CAG | 145 | AGA | AAC |
| 38 | AAG | ACA | 92 | ACG | CAT | 146 | AGA | AAG |
| 39 | AAG | ACC | 93 | ACG | CCA | 147 | AGA | AAT |
| 40 | AAG | ACG | 94 | ACG | CCT | 148 | AGA | ACA |
| 41 | AAG | ACT | 95 | ACG | CGA | 149 | AGA | ACC |
| 42 | AAG | AGC | 96 | ACG | CGT | 150 | AGA | ACG |
| 43 | AAG | AGG | 97 | ACG | CTA | 151 | AGA | ACT |
| 44 | AAG | AGT | 98 | ACG | CTC | 152 | AGA | AGC |
| 45 | ACA | CAA | 99 | ACG | CTG | 153 | AGA | AGG |
| 46 | ACA | CAC | 100 | ACG | CTT | 154 *** | AGA | AGT |
| 47 | ACA | CAG | 101 | ACG | AAC | 155 | AGC | CAA |
| 48 | ACA | CAT | 102 | ACG | AAG | 156 | AGC | CAC |
| 49 | ACA | CCA | 103 | ACG | AAT | 157 *** | AGC | CAG |
| 50 | ACA | CCT | 104 * | ACG | ACA | 158 | AGC | CAT |
| 51 | ACA | CGA | 105 | ACG | ACC | 159 | AGC | CCA |
| 52 | ACA | CGT | 106 | ACG | ACG | 160 | AGC | CCT |
| 53 | ACA | CTA | 107 | ACG | ACT | 161 | AGC | CGA |
| 54 | ACA | CTC | 108 | ACG | AGC | 162 | AGC | CGT |

en ombré : polymorphisme pour une ou plusieurs bandes entre les pools sensible et résistant

* : présence d'une ou plusieurs bandes polymorphes dans le pool sensible

** : présence d'une ou plusieurs bandes polymorphes dans le pool résistant

*** : présence d'une ou plusieurs bandes polymorphes dans le pool sensible et le pool résistant

## TABLEAU 7 suite

| N° de combinaison | Amorce EcoRI | Amorce Msel | N° de combinaison | Amorce EcoRI | Amorce Msel | N° de combinaison | Amorce EcoRI | Amorce Msel |
|---|---|---|---|---|---|---|---|---|
| 163 | AGC | CTA | 218 | AGT | AGC | 273 | CAT | CTA |
| 164 | AGC | CTC | 219 | AGT | AGG | 274 | CAT | CTC |
| 165 | AGC | CTG | 220 | AGT | AGT | 275 | CAT | CTG |
| 166 | AGC | CTT | 221 | ATC | CAA | 276 | CAT | CTT |
| 167 | AGC | AAC | 222 | ATC | CAC | 277 | CAT | AAC |
| 168 | AGC | AAG | 223 | ATC | CAG | 278 | CAT | AAG |
| 169 | AGC | AAT | 224 | ATC | CAT | 279 | CAT | AAT |
| 170 | AGC | ACA | 225 | ATC | CCA | 280 | CAT | ACA |
| 171 | AGC | ACC | 226 | ATC | CCT | 281 | CAT | ACC |
| 172 | AGC | ACG | 227 | ATC | CGA | 282 | CAT | ACG |
| 173 | AGC | ACT | 228 | ATC | CGT | 283 | CAT | ACT |
| 174 | AGC | AGC | 229 | ATC | CTA | 284 | CAT | AGC |
| 175 | AGC | AGG | 230 | ATC | CTC | 285 | CAT | AGG |
| 176 | AGC | AGT | 231 | ATC | CTG | 286 | CAT | AGT |
| 177 | AGG | CAA | 232 | ATC | CTT | 287 | CTA | CAA |
| 178 | AGG | CAC | 233 | ATC | AAC | 288 | CTA | CAC |
| 179 | AGG | CAG | 234 | ATC | AAG | 289 | CTA | CAG |
| 180 | AGG | CAT | 235 | ATC | AAT | 290 | CTA | CAT |
| 181 | AGG | CCA | 236 | ATC | ACA | 291 | CTA | CCA |
| 182 | AGG | CCT | 237 | ATC | ACC | 292 | CTA | CCT |
| 183 | AGG | CGA | 238 | ATC | ACG | 293 | CTA | CGA |
| 184 | AGG | CGT | 239 | ATC | ACT | 294 | CTA | CGT |
| 185 | AGG | CTA | 240 | ATC | AGC | 295 | CTA | CTA |
| 186 | AGG | CTC | 241 | ATC | AGG | 296 | CTA | CTC |
| 187 | AGG | CTG | 242 | ATC | AGT | 297 | CTA | CTG |
| 188 | AGG | CTT | 243 | CAA | CAA | 298 | CTA | CTT |
| 189 | AGG | AAC | 244 | CAA | CAC | 299 | CTA | AAC |
| 190 | AGG | AAG | 245 | CAA | CAG | 300 | CTA | AAG |
| 191 | AGG | AAT | 246 | CAA | CAT | 301 | CTA | AAT |
| 192 | AGG | ACA | 247 | CAA | CCA | 302 | CTA | ACA |
| 193 | AGG | ACC | 248 | CAA | CCT | 303 | CTA | ACC |
| 194 | AGG | ACG | 249 | CAA | CGA | 304 | CTA | ACG |
| 195 | AGG | ACT | 250 | CAA | CGT | 305 | CTA | ACT |
| 196 | AGG | AGC | 251 | CAA | CTA | 306 | CTA | AGC |
| 197 | AGG | AGG | 252 | CAA | CTC | 307 | CTA | AGG |
| 198 | AGG | AGT | 253 | CAA | CTG | 308 | CTA | AGT |
| 199 | AGT | CAA | 254 | CAA | CTT | 309 | CTT | CAA |
| 200 | AGT | CAC | 255 | CAA | AAC | 310 | CTT | CAC |
| 201 | AGT | CAG | 256 | CAA | AAG | 311 | CTT | CAG |
| 202 | AGT | CAT | 257 | CAA | AAT | 312 | CTT | CAT |
| 203 | AGT | CCA | 258 | CAA | ACA | 313 | CTT | CCA |
| 204 | AGT | CCT | 259 | CAA | ACC | 314 | CTT | CCT |
| 205 | AGT | CGA | 260 | CAA | ACG | 315 | CTT | CGA |
| 206 | AGT | CGT | 261 | CAA | ACT | 316 | CTT | CGT |
| 207 | AGT | CTA | 262 | CAA | AGC | 317 | CTT | CTA |
| 208 | AGT | CTC | 263 | CAA | AGG | 318 | CTT | CTC |
| 209 | AGT | CTG | 264 | CAA | AGT | 319 | CTT | CTG |
| 210 | AGT | CTT | 265 | CAT | CAA | 320 | CTT | CTT |
| 211 | AGT | AAC | 266 | CAT | CAC | 321 | CTT | AAC |
| 212 | AGT | AAG | 267 | CAT | CAG | 322 | CTT | AAG |
| 213 | AGT | AAT | 268 | CAT | CAT | 323 | CTT | AAT |
| 214 | AGT | ACA | 269 | CAT | CCA | 324 | CTT | ACA |
| 215 | AGT | ACC | 270 | CAT | CCT | 325 | CTT | ACC |
| 216 | AGT | ACG | 271 | CAT | CGA | 326 | CTT | ACG |
| 217 | AGT | ACT | 272 | CAT | CGT | 327 | CTT | ACT |
| | | | | | | 328 | CTT | AGT |

en ombre : polymorphisme pour une ou plusieurs bandes entre les pools sensible et résistant

* : presence d'une ou plusieurs bandes polymorphes dans le pool sensible

** : présence d'une ou plusieurs bandes polymorphes dans le pool résistant

Ce criblage a permis d'identifier une ou plusieurs bandes polymorphes selon leur apparition chez le parent sensible et/ou le parent résistant. 23 couples d'amorces ont permis d'identifier un polymorphisme entre les parents confirmés par les pools d'ADN F2 sensible ou résistant.Le tableau récapitule et donne la position dans l'intervalle M1-M2 des marqueurs AFLP liés au locus de résistance élevée au virus de la panachure jaune du riz.

## TABLEAU 8

| N° de combinaison | Nucléotides variables | | Présence de(s) bande(s) | | Position des marqueurs sur l'intervalle M1-M2 |
|---|---|---|---|---|---|
| | Amorce EcoRI | Amorce MseI | pool sensible | pool résistant | |
| 3 | AAC | CAG | + | - | = Marqueur cloné M1 |
| 69 | ACC | CAG | + | - | = Marqueur cloné M2 |
| 77 | ACC | CTG | - | - | non déterminé |
| 81 | ACC | AAT | - | + | non déterminé |
| 86 | ACC | AGC | - | + | non déterminé |
| 91 | ACG | CAG | - | + | non déterminé |
| 104 | ACG | ACA | + | - | entre R et Rm273 |
| 154 | AGA | AGT | + | + | au delà de M2 |
| 157 | AGC | CAG | - | + | en coségrégation avec M2 |
| 174 | AGC | AGC | - | + | non déterminé |
| 175 | AGC | AGC | + | + | entre M1 et Rm241 |
| 197 | AGG | AGG | + | + | entre M1 et Rm241 |
| 215 | AGT | ACC | - | + | non déterminé |
| 220 | AGT | AGT | + | - | entre Rm273 et M2 |
| 233 | ATC | AAG | + | + | entre M1 et Rm241 |
| 250 | CAA | OGT | - | - | non déterminé |
| 254 | CAA | CTT | + | - | au delà de M2 |
| 258 | CAA | ACA | + | - | entre M 1 et Rm241 |
| 280 | CAT | ACA | + | - | au delà de M2 |
| 287 | CTA | CAA | + | - | entre Rm273 et M2 |
| 291 | CTA | CCA | + | - | entre M 1 et Rm241 |
| 318 | CTT | CTC | + | + | entre Rm273 et M2 |
| 319 | CTT | CTG | - | + | non déterminé |

Après vérification individuelle sur chacun des individus composant les pools, les marqueurs candidats correspondant à des bandes présentes chez le parent IR64 peuvent être testées sur les recombinants identifiés dans l'exemple 11. 9 marqueurs ont été confirmés ainsi comme appartenant à l'intervalle M1 -M2. Le tableau 9 donne l'ordonnancement dans l'intervalle M1-M2 des marqueurs AFLP identifiés par comparaison de pools d'ADN sensible et résistant à partir d'une sous population résistante d'une F2 (IR64 x Gigante).

## TABLEAU 9

| Individus résistants F2 (IR64 x Gigante) | M1 | E-AGG M-AGG | E-ATC M-AAG | E-CAA M-ACA | E-AGC M-AGG | E-CTA M-CCA | RM241 | RM252 | Résist. RYMV | E-ACG M-ACA | RM273 | E-AGT M-AGT | E-CTT M-CTC | E-CTA M-CAA | M2 |
|---|---|---|---|---|---|---|---|---|---|---|---|---|---|---|---|
| 2 | H | D | D | D | D | D | . | B | B | B | B | B | B | B | B |
| 7 | H | D | D | D | D | D | . | B | B | B | B | B | B | B | B |
| 8 | H | D | D | D | D | D | . | B | B | B | B | B | B | B | B |
| 10 | H | D | D | D | E | D | . | B | B | B | B | B | B | B | B |
| 21 | H | D | D | D | D | B | C | B | B | B | B | B | B | B | B |
| 23 | H | D | D | D | E | D | . | B | B | B | B | B | B | B | B |
| 25 | H | D | D | D | D | D | E | H | B | B | B | B | B | B | B |
| 29 | H | D | D | D | B | B | C | B | B | B | B | B | B | B | B |
| 37 | H | D | D | D | E | D | E | H | B | B | B | B | B | B | B |
| 48 | H | D | D | D | D | D | . | B | B | B | B | B | B | B | B |
| 55 | H | D | D | D | D | D | E | H | B | B | B | B | B | B | B |
| 61 | H | D | D | D | D | D | E | H | B | B | B | B | B | B | B |
| 65 | H | D | D | D | . | B | C | B | B | B | B | B | B | B | B |
| 95 | H | E | D | D | D | B | C | B | B | B | B | B | B | B | B |
| 103 | H | E | D | D | . | B | C | B | B | B | B | B | B | B | B |
| 104 | H | D | D | D | B | B | C | B | B | B | B | B | B | B | B |
| 109 | H | B | B | B | B | B | C | B | B | B | B | B | B | B | B |
| 111 | H | E | D | D | D | D | . | B | B | B | B | B | B | B | B |
| 119 | H | D | D | D | D | D | . | B | B | B | B | B | B | B | B |
| 120 | A | D | D | D | D | B | C | B | B | B | B | B | B | B | B |
| 126 | H | E | E | E | E | D | . | B | B | B | B | B | B | B | B |
| 127 | H | . | . | . | . | B | C | B | B | B | B | B | B | B | B |
| 131 | H | E | E | E | E | D | . | B | B | B | B | B | B | B | B |
| 133 | H | . | . | . | . | B | C | B | B | . | B | B | B | B | B |
| 141 | H | E | E | E | E | D | E | H | B | B | B | B | B | B | B |
| 154 | H | E | E | E | E | D | . | B | B | B | B | B | B | B | B |
| 159 | H | E | E | E | E | D | . | B | B | B | B | B | B | B | B |
| 159 | H | . | . | . | . | B | C | B | B | . | B | B | B | B | B |
| 160 | H | E | E | E | E | D | . | B | B | B | B | B | B | B | B |
| 161 | H | E | E | E | E | D | . | B | B | B | B | B | B | B | B |
| 163 | H | . | . | . | . | B | C | B | B | B | B | B | B | B | B |
| 167 | H | . | . | . | . | B | B | B | B | B | B | B | B | B | B |
| 171 | H | . | . | . | . | B | B | B | B | B | B | B | B | B | B |
| 175 | H | E | E | E | D | D | B | B | B | B | B | B | B | B | B |
| 179 | H | . | . | . | B | B | B | B | B | B | B | B | B | B | B |
| 183 | H | E | E | E | E | D | B | B | B | B | B | B | B | B | B |
| 35 | H | D | D | D | D | D | H | H | B | D | H | D | D | D | D |
| 135 | H | E | E | E | E | D | H | H | B | B | H | D | D | D | D |
| 17 | H | B | B | B | B | B | . | B | B | B | H | D | D | D | D |
| 20 | B | B | B | B | B | B | B | B | B | D | H | D | D | D | D |
| 38 | B | B | B | B | B | B | . | B | B | D | H | D | D | D | D |
| 93 | B | B | B | B | B | B | B | B | B | D | H | D | D | D | D |
| 105 | B | B | B | B | B | B | B | B | B | B | H | D | D | D | D |
| 145 | B | . | . | . | B | B | B | B | B | B | B | B | B | B | D |
| 160 | B | . | . | B | B | B | B | B | B | B | B | D | D | D | D |

Fréquence d'individus recombinés*

| | | | | | | | | | | | | | | | |
|---|---|---|---|---|---|---|---|---|---|---|---|---|---|---|---|
| Intervalle M1 - R | | 0,97 | 0,97 | 0,97 | 0,87 | 0,61 | 0,29 | 0,13 | | | | | | | |
| Intervalle R - M2 | | | | | | | | | | 0,67 | 0,78 | 0,89 | 0,89 | 0,89 | |
| Distance / résistance (cM) | | 11,4** | 11,03 | 11,03 | 11,03 | 9,88 | 6,90 | 3,33 | 2,10 | 0,00 | 3,33 | 3,89 | 4,44 | 4,44 | 4,44 / 5,0** |

A : génotype homozygote pour l'allèle du parent sensible (IR64)

H : génotype hétérozygote

B : génotype homozygote pour l'allèle du parent résistant ( Gigante)

D : génotype non homozygote pour l'allèle du parent résistant (gigante)

\* . sous l'hypothèse d'absence de double recombinaison dans l'intervalle M1 - R et M2 - R

\*\* : distance estimée à partir de la cartographie de la résistance sur 183 F2 (IR64 x Gigante) cf (figure X)

14 bandes provenant du parent résistant ont été également identifiées et seront confirmées ou non sur les recombinants générés dans la population F2 (IR64 x Gigante).

**Exemple 13 :** Ancrage du locus de résistance au RYMV à l'aide de marqueurs microsatellites

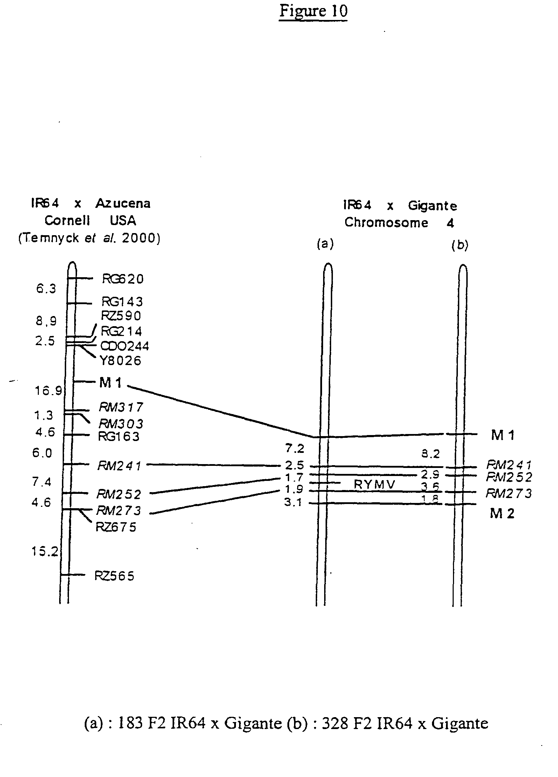

Le marqueur M1 ayant été placé sur le chromosome 4 de la carte génétique (IR64 x Azucena ; exemple 9), des marqueurs microsatellites tel que définis dans (6) et appartenant à ce chromosome ont été utilisés pour affiner la cartographie du locus de résistance au RYMV. Les marqueurs microsatellites suivants ont été testés : RM241, RM252 (1), RM273 et RM177 (6) dans les conditions expérimentales définies dans (1) et (6). A l'exception du marqueur RM177 non polymorphe entre les parents IR64 et Gigante, les marqueurs RM241, RM252, RM273 ont été cartographiés sur une population F2 (IR64 x Gigante) évaluée parallèlement pour la résistance au RYMV. Les résultats sur 183 individus F2 permettent de caractériser un intervalle d'environ 3.6 cM borné par les deux locus microsatellites RM 252 et RM273 encadrant le gène de résistance au RYMV.(voir figure 10 (a))

**Exemple 14 :** Cartographie fine de l'intervalle portant le locus de résistance et ordonnancement des marqueurs de résistance dans l'intervalle M1-M2

Les 45 individus F2 (IR64 x Gigante) résistants et recombinants pour les marqueurs M1 et M2 ont été caractérisés pour les marqueurs microsatellites identifiés dans l'exemple 13. La cartographie des marqueurs en ségrégation sur la totalité des individus F2 (IR64 x Gigante) disponibles (321) confirme l'ordre et la distance entre les marqueurs de l'intervalle M1 - M2 et en particulier l'intervalle RM252 - RM273 qui est évalué à 3.6 cM (figure 10(b)). Les 45 individus F2 (IR64 x Gigante) résistants et recombinants pour les marqueurs M1 et M2 permettent de confirmer l'ordre des marqueurs AFLP identifiés dans l'exemple 12. Un marqueur AFLP EACG/MACA reste compris dans l'intervalle RM252 - RM273 et représente le marqueur le plus proche du locus de résistance au RYMV (Tableau 9). Au total sur les 321 individus F2 testés, il reste 20 individus recombinés d'un côté ou de l'autre du locus de résistance au RYMV et qui pourront être avantageusement utilisés pour identifier des marqueurs plus proches et/ou cloner le gène de résistance.

**Exemple 15 :** Transfert de la résistance assistée par les marqueurs

Les marqueurs proches du locus de résistance ont été testés sur des variétés irriguées très sensibles au virus du RYMV (var. BG90-2, Bouaké189, Jaya). 3 marqueurs (M1, RM241, RM252) présentent un polymorphisme entre ces 3 variétés et la variété Gigante ce qui permet d'envisager l'utilisation de ces marqueurs pour transférer la résistance dans des génotypes sensibles. Le transfert expérimental de la résistance dans ces variétés a été réalisé jusqu'au niveau du 2eme recroisement. A chaque croisement, les plantes sont vérifiées pour la présence des marqueurs en provenance de la variété Gigante et la ségrégation pour la résistance est contrôlée par des tests de descendance sur F2. Les résultats sont donnés dans le tableau 10.

**TABLEAU 10**

| parent récurent | Polymorphisme / parent donneur (Gigante) | | | | | Génération obtenue | % théorique parent récurent | Nb de lignées obtenues |
|---|---|---|---|---|---|---|---|---|
| | M1 | RM241 | RM252 | RM273 | RM177 | | | |
| BG90-2 | poly | poly | poly | - | - | BC2F2 | 87,5 | 4 |
| Bouaké 189 | poly | poly | poly | - | - | BC2F2 | 87,5 | 1 |
| Jaya | poly | poly | poly | - | - | BC2F2 | 87,5 | 2 |
| IR64 | poly | poly | poly | poly | mono | BC3 | 93,7 | 5 |

Exemple 16 : Utilisation du virus RYMV ou du complexe virus/ribonucléoprotéines comme appâts pour capturer les protéines cibles indispensables au cycle infectieux du RYMV.

I Caractérisation *in vitro* et *in vivo* de trois isoformes du RYMV calcium et pH indépendantes

Trois isoformes sont décrites en utilisant la chromatographie échangeuse d'ions, le principe de cette séparation est basé sur la stabilité des particules.
Les formes compactes sont les plus stables car elles sont bloquées par les ions divalents calcium rendant la particule insensible au pH. Cette forme n'accroche pas la résine échangeuse d'ions et traverse la colonne sans dommage. Les formes transitionnelles sont décrites pour la première fois et résultent de particules compactes ou de particules gonflées. Ces particules sont sans calcium, ce qui les rend sensibles au pH. Ainsi, à pH acide, elles sont gardées compactes, alors qu'à pH basique elles sont gonflées. On peut différencier ces deux isoformes ( compactes et transitionnelles) par modification du pH dans le tampon de chromatographie. A pH basique, les particules transitionnelles instantanément gonflent et explosent dans la colonne, car elles ne sont pas suffisamment stables pour supporter la pression du chromatographe (environ 1000 à 1500 psi) pression. La protéine de capside, qui résulte de cette dissociation, s'accroche sur la résine échangeuse d'ions. Les formes compactes sont purifiées ainsi à pH basique. Les formes gonflées sont très difficiles à isoler du fait de leur instabilité, mais peuvent être produites en présence d'un chélateur d'ions divalents (EDTA ou EGTA) et à pH basique à partir de particules compactes. Ainsi, après ce traitement, toutes les particules deviennent instables et explosent dans la colonne.

Conformément à l'invention, pour capturer les protéines cibles, on utilise :

**I** La particule compacte obtenue après dialyse dans 10 mM acétate de sodium pH 5,0 et $CaCl_2$ 3 mM, environ 6 heures et purification par chromatographie à pH 8,0. Protéines cibles isolées après extraction du virus des plantes infectées ou après exposition des particules compactes (purifiées par Biocad) et protéines extraites de suspension cellulaire d'une variété sensible au virus (IR64).

**II** La particule transitionnelle est obtenue à partir de la particule compacte en présence d'EDTA et à pH acide.

**III** La particule gonflée et le complexe ribonucléique sont obtenus à partir des particules transitionnelles à pH basique. A ce pH, le complexe ribonucléoprotéique est obtenu spontanément à partir des particules gonflées (très instables).

II /Méthode pour capturer les protéines cibles

-A partir de virus extrait de plantes sensibles (IR64) infectées.

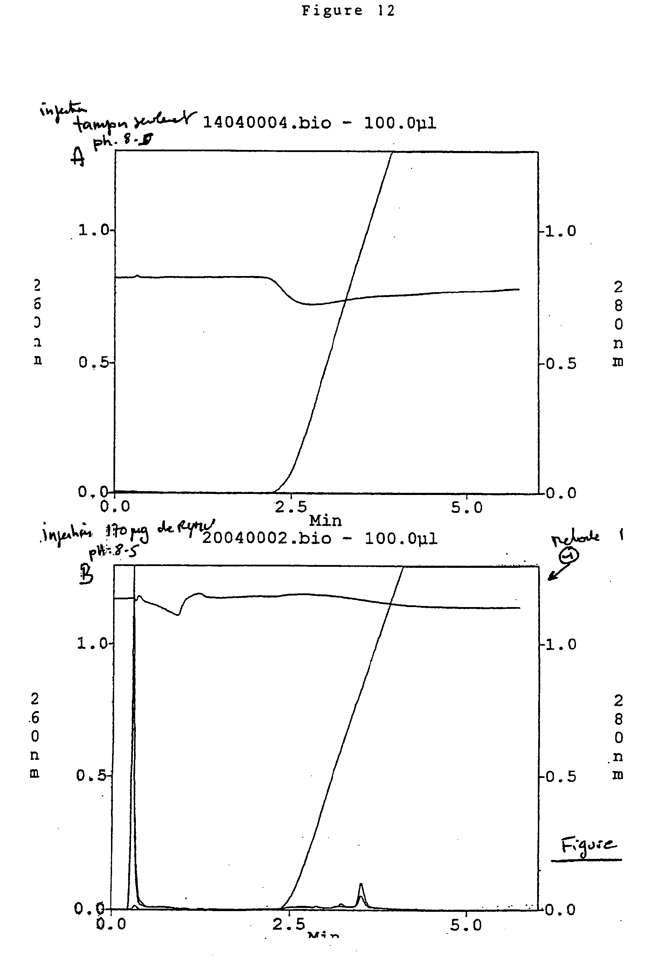

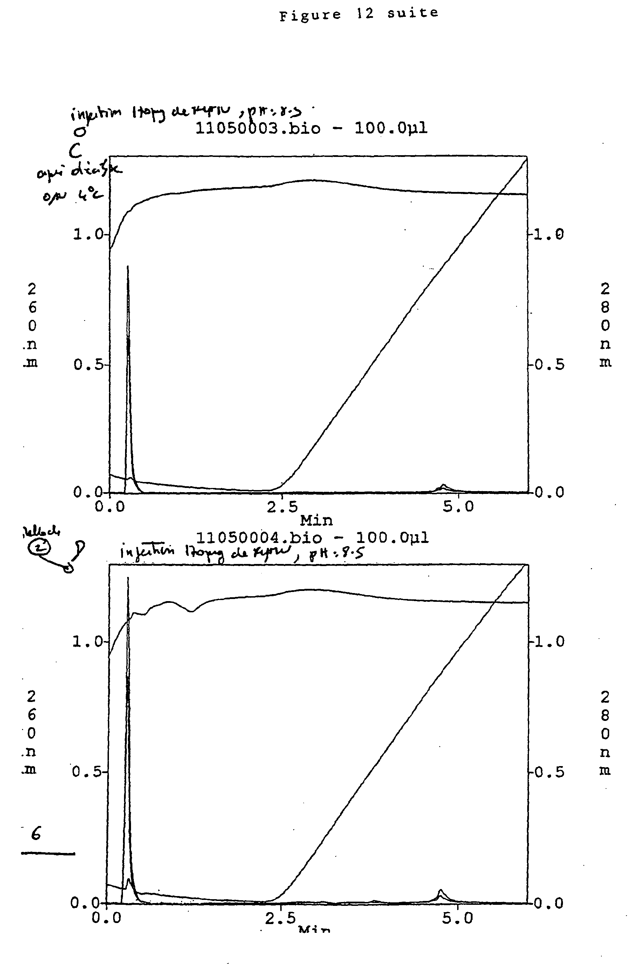

Les résultats sont présentés en figure 12. Fig. 12A: chromatogramme contrôle sans injection à pH 8,5; Fig. 12B:chromatogramme après injection de 100 $\mu$l de virus à 1.7 $\mu$g/$\mu$l, pH 8,5, méthode 1 (gradient NaCl de 0 à 2550 mM), Fig. 12C: chromatogramme après injection de 100 $\mu$l de virus dialysé durant environ 14h dans 10 mM acétate de sodium pH 5 et 3 mM $CaCl_2$ ; Figure 12D: chromatogramme après injection de 100 $\mu$l de virus (non dialysé) à 1.7 $\mu$g/ $\mu$l, pH 8.5, méthode 2 (gradient NaCl de 0 à 1500 mM et 1500 à 2550 mM, voir méthode après les exemples). Récolte des fractions (1ml), précipitation acétone 800$\mu$l, 2 heures à 4 °C, centrifugation 20 minutes 13000tr/mn, culot séché au speed-vack 5 min., puis remis en suspension dans environ 40 $\mu$l de tampon Tris-base 10 mM, pH 7,4. Les échantillons sont conservés au congélateur à -20°C.

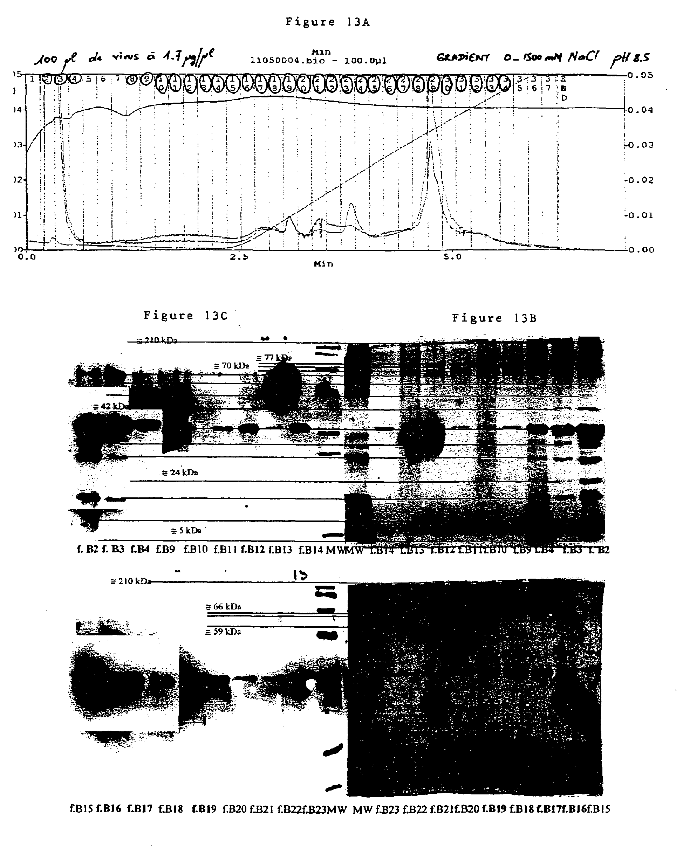

Après injection de 170 $\mu$g de virus dans le Biocad à pH : 8,0, méthode 1 (Figure 13A), les différentes fractions sont récoltées, précipitées à l'acétone, reprise dans un tampon Tris-base 10 mM, pH 7,4 et déposées sur mini-gel SDS-PAGE puis le gel est révélé au nitrate d'argent (Biorad) (Figure 13B) et Westernblot (Figure 13C) en utilisant un anticorps monoclonal non discriminant MabE.5 (Denis Fargette ,IRD) anti -protéine de capside.

Les bandes non immunodétectées correspondent à des protéines de plante potentiellement intéressantes. Il s'agit de protéines de_5, 24, 42, 49, 59, 66, 70, 77 et 210 kDa

-A partir de virus extrait, dialysé (durant environ 14h dans 10 mM acétate de sodium pH 5,0 et $CaCl_2$ 3 mM), puis purifié sur Biocad = fraction 2 (A2), le virus est mis en contact avec les protéines de suspension cellulaire de riz (IR 64) pour piéger des protéines cibles.

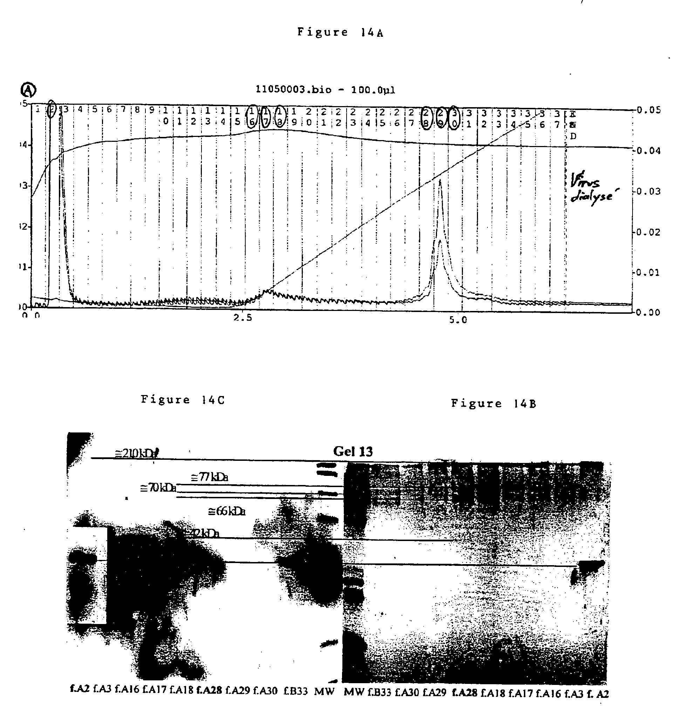

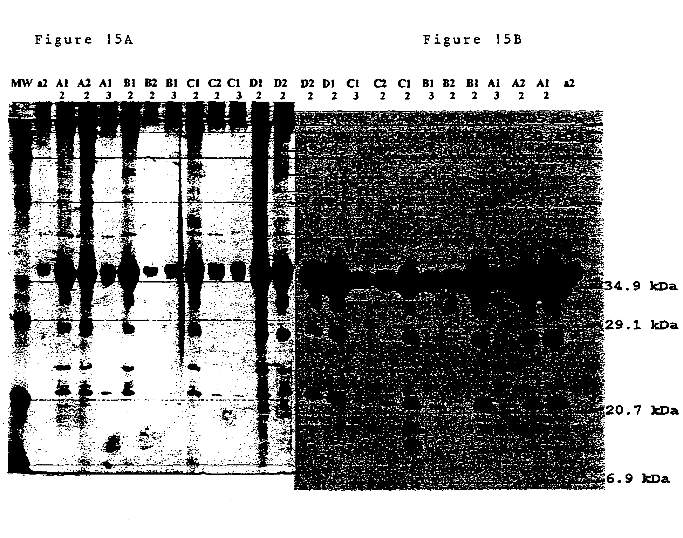

Purification et vérification de la pureté de la fraction 2 (Fig. 14A). Dans la fraction 2, le virus est pur car il n'y a plus de présence de sous-bandes détectées après un gel de SDS-PAGE et révélation au nitrate d'argent (Fig. 14B et 14C, fraction A2 à comparer avec B2 dans les Fig. 13B et 13C). Après une incubation de 350 $\mu$g de virus et 860 $\mu$g de protéines extraites de suspension cellulaire (IR64) à 4°C, pendant 12 heures, dans différentes solutions d'incubation (Fig 15), les échantillons sont extraits, avec ou sans NaCl. Les échantillons sont ensuite purifiés sur Biocad et la fraction virale après précipitation à l'acétone (fraction 2 et 3); est analysée sur grand gel SDS-PAGE coloré au nitrate d'argent (Fig 15A) et immunodétection avec le Mab E.5 (Fig 15B).

Les protéines cibles (non immunodétectées par le Mab E.5) ont des poids moléculaires similaires à celles isolées des feuilles infectées ; elles sont particulièrement visibles dans les conditions D2 (pH 8,0 avec 10 mM DTT et 0,3 M de NaCl): il s'agit des protéines de 24, 45, 51, 57, 63, 85 et des protéines situées au-dessus de 120 kDa.

III/ Clonage des protéines cibles et utilisation pour l'identification d'une nouvelle classe de gènes de résistance

Les protéines cibles isolées sont séquencées dans leur partie N-terminale (protéines 24, 42-45 kDa , protéines aux environs de 57, 63 et 85 kDa, et les protéines supérieures à 120kDa). On identifie des amorces 5' dégénérées. Le clonage des ADNc est effectué dans les banques des variétés indica (IR64 et Gigante), japonica tempéré (02428), japonica tropical (Azucena) et glaberrima (TOG 5681 et TOG 5673). On procède ensuite à l'analyse des séquences en utilisant les techniques classiques (homologie, fonction putative et polymorphisme).

IV/Matériels et Méthodes

Extraction du virus: L'extraction est réalisée à l'aide des étapes consistant à : récolter des feuilles fraîches (ou conservées à -80°C) de plantes infectées par le virus (les symptômes doivent être prononcés). La durée d'infection dépend de la variété de riz utilisée (les variétés tolérantes permettent d'obtenir une plus grande quantité de virus).

- Broyer les feuilles dans l'azote liquide pour obtenir une poudre fine, et conserver au froid.
- Ajouter le <u>tampon d'extraction</u> (Acétate de sodium* 0,1 M pH : 5,0) additionné de β-mercaptoéthanol à 0.2% (≈1 litre de tampon pour 100 g de feuilles broyées) et agiter pendant 2 mn.
- Filtrer la suspension obtenue avec un tissu de type « cheesecloth » et presser à la main le culot restant dans le tissu.
- Ajouter 1 volume de chloroforme et agiter pendant 2 mn.
- Centrifuger à 10000g pendant10 mn à 4°C. et récupérer la phase aqueuse supérieure.
- Evaluer le volume de la phase aqueuse et ajouter du NaCl pour une concentration finale 0.3M. Laisser dissoudre (en chambre froide) et ajouter du PEG8000 pour une concentration finale de 6%.
- Laisser sous agitation pendant la nuit. (précipitation des virus)
- Centrifuger la suspension pendant 30mn à 22000 g à 4°C dans des pots de 250ml.
- Jeter le surnageant et sécher quelques minutes à l'air le culot de virus. Reprendre le culot de chaque pot par 1ml de <u>tampon d'extraction</u> (1) et remettre en suspension au froid.
- Réunir les fractions obtenues et rincer les pots avec 0.5ml de tampon que l'on rajoute aux fractions précédentes.
- Centrifuger 10 mn à 12000g pour éliminer les impuretés solides et récupérer le surnageant.
- Doser, après dilution, au spectrophotomètre à 259 nm et calculer la concentration du virus :

$$\frac{D.O.259 \times dilution}{6.5} \; en \; \mu g/\mu l$$

où 6,5 correspond à la D.O. 259 d'une suspension virale à 1 $\mu$g/$\mu$l.

Un rapport $\dfrac{D.O.259}{D.O.280} \geq 1,5$ montre une bonne purification du virus, sinon effectuer une purification par gradient de sucrose.

* Solution A : Acide acétique 2M (115,5 dans 1000ml) et solution B : acétate de sodium 2M (164 g de $C_2H_3O_2$ ou 272 g de $C_2H_3O_2Na$, 3$H_2O$ dans 1000 ml. 14,8 ml de solution A + 35,2 ml de solution B dans 1000 ml total, ajouter d'abord la solution B, puis la solution A progressivement jusqu'à l'obtention du pH 5 ; ajuster le pH si nécessaire avec de la solution A.

Chromatographie échangeuse d'ions

Chromatographe PerSeptive Biosystems BIOCAD 700E (Framingham, MA, USA) avec un collecteur de fraction Gilson F-250. Des échantillons de 100 $\mu$l de virus sont injectés et purifiés sur une colonne anionique échangeuse d'ions POROS prepacked (HQ/H, 4.6 mm D / 100 mm L, CV= 1.7 ml), colonne contenant des particules de 10 $\mu$m POROS. Le tampon d'élution utilisé est composé de 15 mM Bis-Tris Propane : Tris base (50/50, Sigma) à pH 6 ou pH 8,5 à un débit de 6 ml/min, pour une pression interne d'environ 1500 psi. Avant injection, la colonne est équilibrée avec le tampon d'élution. Après injection du virus, la colonne est lavée avec du tampon d'élution, puis par un gradient de NaCl de 0 à 2,55 M pendant 3 min (méthode 1) ou par un gradient de NaCl de 0 à 1550 mM pendant 4 minutes puis un gradient de 1500 mM à 2550 mM pendant 30 secondes (méthode 2). Enfin une dernière étape (environ 1 minute) avec le tampon d'élution et une concentration de NaCl à 2550 mM est effectuée. L'absorbance est enregistrée à 260 et 280 nm. Après 3 injections du même échantillon, le maximum de variation observé est d'environ 3%. Le niveau minimum de détection pour le virus est d'environ 7 ng/$\mu$l. Les fractions de 1 ml sont récoltées et sont dialysées durant environ 14h à 4 °C dans le tampon 20 mM phosphate avant injection. Les échantillons sont examinés par microscopie électronique.

Suspension cellulaire

Les graines sont décortiquées, puis mises à désinfecter dans de l'éthanol 70 (v/v) pendant 1 minute, et ensuite dans une solution d'hypochlorite de sodium à 2,6% en faisant un vide partiel pour un meilleur contact pendant 45 mn. Après 4 lavages à l'eau stérile, les graines sont déposées sur le milieu gélosé (milieu MS) dans les boites de Pétri (fermées avec du parafilm) et mises à l'obscurité à 25°C.

Six semaines plus tard, les cals développés à partir des embryons sont mis en cultures dans du milieu liquide utilisé pour la multiplication cellulaire (plusieurs formations globuleuses provenant de plusieurs cals, par Erlen). Trois semaines plus tard, les cals mis en culture en milieu liquide ont proliféré, et les suspensions sont repiquées. Ensuite les suspensions sont repiquées tous les 10 -15 jours et les protéines extraites.

MILIEUX DE CULTURE POUR SUSPENSIONS CELLULAIRES DE RIZ

Milieu SZ (Zhang *and al.* 1998)

| MACRO ELEMENTS X 10 (pour 1 litre) | |
| --- | --- |
| *100 ml de solution mère par litre de solution finale* | |
| $KNO_3$ | 40,00 g |
| $(NH_4)_2SO_4$ | 3,30 g |
| $MgSO_4,7H_2O$ | 2,46 g |
| $NaH_2PO4$ | 2,76 g ($2H_2O$ : 3,12g - anhydre : 2,40 g) |
| $CaCl_2,2H_2O$ | 1,47 g |

| B5 MICRO ELEMENTS X 100 (pour 1 litre) | |
| --- | --- |
| *10 ml de solution* | *mère par litre de solution finale* |
| $MnSO_4, 7H_2O$ | 1349 mg |
| $ZnSO_4$ | 112 mg ($7H_2O$ : 200 mg) |
| KI | 75 mg |
| $Na_2MoO_4, 2H_2O$ | 25 mg |
| $H_3BO_3$ | 300 mg |
| $CuSO_4, 5H_2O$ | 2,5 mg |
| $CoCl_2, 6H_2O$ | 2,5 mg |

| B5 VITAMINESX 1000 (pour 100 millilitres) | | |
| --- | --- | --- |
| *1 ml de solution mère par litre de solution finale* | | |
| Acide nicotinique | 100 mg | |
| Thiamine-HCl | 1000 mg | |
| Pyridoxine-HCl | 100 mg | |
| Myo-Inositol | 10 g | |
| Proline | | 500 mg/litre final |
| Glutamine | | 500 mg/litre final |
| Hydrolysat enzymatique de caséine | | 300 mg/litre final |

| Fer EDTA X 1000 (pour 1 litre) | |
| --- | --- |
| *1 ml de solution mère par litre de solution finale* | |
| Sinon $FeSO_4, 7H_2O$ | 2,8 g |
| $Na_2EDTA$ | 3,7 g |

<u>HORMONE 2,4-D</u> X 1000
*1 ml de solution mère par litre de solution finale*
Solution stock à 2 mg/ml
<u>MALTOSE</u> ET <u>SUCROSE</u> (POUR 2428) à 30g/l
<u>pH5.8</u>
MILIEUX D'INDUCTION DE LA CALLOGENESE DU RIZ

M.S. (Murashige T. & Skoog F. 1962)

| MACRO ELEMENTSX 10 | (pour 1 litre) |
|---|---|
| 100 ml de solution mère par litre de solution finale | |
| $KNO_3$ | 19,00 g |
| $NH_4NO_3$ | 16,50 g |
| $MgSO_4,7H_2O$ | 3,70 g |
| $KH_2PO4$ | 1,70 g |
| $CaCl_2,2H_2O$ | 4,40 g |

| MICRO ELEMENTS X 100 (pour 1 litre) | |
|---|---|
| 10 ml de solution mère par litre de solution finale | |
| $MnSO_4,H_2O$ | 1690 mg |
| $ZnSO_4, 7H_2O$ | 860 mg |
| KI | 83 mg |
| $Na_2MoO_4, 2H_2O$ | 25 mg |
| $H_3BO_3$ | 620 mg |
| $CuSO_4, 5H_2O$ . | 2,5 mg |
| $CoCl_2, 6H_2O$ | 2,5 mg |

| VITAMINESX 1000 (pour 100 millilitres) | |
|---|---|
| 1 ml de solution mère par litre de solution finale | |
| Acide nicotinique | 50 mg |
| Thiamine-HCl | 10 mg |
| Pyridoxine-HCl | 50 mg |
| Myo-Inositol | 10 g |
| Glycine | 200 mg |

| Fer EDTA X1000 | (pour 1 litre) |
|---|---|
| 1 ml de solution mère par litre de solution finale | |
| Sinon $FeSO_4, 7H_2O$ | 2,8 g |
| $Na_2EDTA$ | 3,7 g |

HORMONE 2.4-D X 1000
1 ml de solution mère par litre de solution finale
Solution stock à 2 mg/ml
MALTOSE OU SUCROSE à 30g/l
pH 5,8
PHYTAGEL 2.5 g/litre
NB (Milieu d'induction et de subculture de cals)

| N6 MACRO ELEMENTS X 10 | (pour 1 litre) |
|---|---|
| 100 ml de solution mère par litre de solution finale | |
| $KNO_3$ | 28,30 g |
| $(NH_4)_2SO_4$ | 4,63 g |
| $MgSO_4,7H_2O$ | 1,40 g |
| $KH_2PO4$ | 4,00 g |
| $CaCl_2,2H_2O$ | 1,65 g |

| B5 MICRO ELEMENTS X 100 | (pour 1 litre) |
| --- | --- |
| **10 ml de solution mère par litre de solution finale** | |
| $MnSO_4, 7H_2O$ | 1349 mg |
| $ZnSO_4$ | 112 mg ($7H_2O$ : 200 mg) |
| KI | 75 mg |
| $Na_2MoO_4, 2H_2O$ | 25 mg |
| $H_3BO_3$ | 300 mg |
| $CuSO_4, 5H_2O$ | 2,5 mg |
| $CoCl_2, 6H_2O$ | 2,5 mg |

| B5 VITAMINES X 1000 (pour 100 millilitres) | |
| --- | --- |
| **1 ml de solution mère par litre de solution finale** | |
| Acide nicotinique | 100 mg |
| Thiamine-HCl | 1000 mg |
| Pyridoxine-HCl | 100 mg |
| Myo-Inositol | 10 g |
| Ou vitamines de Gamborg à 11,2 | |
| Ajouter : concentration finale | |
| Proline | 500 mg/litre final |
| Glutamine | 500 mg/litre final |
| Hydrolysat enzymatique de caséine | 300 mg/litre final |

| Fer EDTA X1000 (pour 1 litre) | |
| --- | --- |
| **1 ml de solution mère par litre de solution finale** | |
| Sinon $FeSO_4, 7H_2O$ | 2,8 g |
| $Na_2EDTA$ | 3,7 g |

Ou EDTA Sel de sodium ferrique (Sigma E-6760) à 4,15 g/l

HORMONE 2.4-D X 1000

*1 ml de solution mère par litre de solution finale*

Solution stock à 2 mg/ml

MALTOSE ET SUCROSE à 30g/l

PHYTAGEL 2,6 g/litre

pH 5. 8

EXTRACTION DES PROTEINES

*EXTRACTION DES PROTEINES DES SUSPENSIONS CELLULAIRES*

• Tampon d'extraction pour suspensions cellulaires:

| | **concentration finale** | **pour 100 ml** |
| --- | --- | --- |
| Tris | 20 mM, pH 7.4 | 50 ml de Tris 40 mM pH7.4 |
| NaCl | 100 mM | 584 mg |
| $Na_2EDTA, 2H_2O$ | 10 mM | 372 mg |
| Glucose | 25 mM | 856 mg |
| SDS (dénaturant) | 0.1% | 0.5 ml de SDS 20% |
| Triton-X-100 (non-dénaturant) | 0.1% | 100 $\mu$l |

# EP 1 185 708 B1

(suite)

| | concentration finale | pour 100 ml |
|---|---|---|
| DNAses et RNAses | 1 µg/ml | |
| Inhibiteurs de protéines | | 2 pastilles ou 4 ml de sol conc. |
| EGTA | 5 mM | 190 mg |
| Glycérol | 5% | 5 ml |
| DTT | 5 mM | 77 mg |

<u>Réajuster le pH à 7.4 avec HCl</u>
- 5 g de suspensions cellulaires sont mises dans un mortier avec du sable de Fontainebleau stérilisé.
- Ajout de 1 ml de tampon et broyage, puis ajout de 4 ml de tampon.
- Les variétés de riz sont extraites ainsi en conditions dénaturantes (tampon avec SDS) et en conditions non-dénaturantes (tampon avec Triton). Centrifugation 15 mn à 15000g à 3°C.
- Récupération et aliquotes de 1ml du surnageant en tube de 1,5ml qui sont immédiatement stockés à -80°C.

Toutes les étapes se font au maximum dans la glace et le plus rapidement possible.

*DOSAGE DES PROTEINES*

- Une gamme étalon de 1000 µg/ml à 100 µg/ml est faite avec de la BSA (2mg/ml) pour chaque tampon dénaturant et non-dénaturant.
- Les 4 échantillons sont dilués 5 fois dans les tampons respectifs.
- A 50 µl de chaque échantillon et point de gamme sont ajoutés 2,5 ml de solution Coomassie® Protein Assay Reagent, puis on mélange.
- Lecture à 595 nm au spectro dans des cuves jetables.

**MODE OPERATOIRE POUR LA REALISATION DE MEMBRANES**

Les gels d'acrylamide (19:1 ou 29:1) sont préparés de la façon suivante:

<u>Mini gel acrylamide 12% SDS-Page</u>

| Running gel à 12% | **Pour 1 gel de 1mm** |
|---|---|
| Acrylamide bis-acrylamide 40% | 1.5 ml |
| Tris-HCl 1.5M pH 8.8 | 1.3 ml |
| SDS 20% | 25 µl |
| Ammonium Persulfate 10% | 50 µl |
| Temed | 5 µl |
| $H_2O$ | 2.2 ml |
| Stacking gel à 5% | **Pour 1 gel de 1mm** |
| Acrylamide bis-acrylamide 40% | 250 µl |
| Tris-HCl 1M pH 6.8 | 250 µl |
| SDS 20% | 10 µl |
| Persulfate d'ammonium 10% | 20 µl |
| Temed | 2 µl |
| $H_2O$ | 1.5 ml |

- Le running gel est coulé jusqu'à 2 cm du haut de la plaque puis recouvert de butamol-1 (facilite la polymérisation en évitant le contact de l'air).
- Après polymérisation(15-30mn), retirer le butanol avec un papier Whatmann, puis couler le stacking gel et mettre en place le peigne.
- Après polymérisation, laver les puits avec le tampon de migration, puis charger les échantillons auparavant dénaturés 5 mn à 98°C avec 1 volume de tampon de charge.

- Faire migrer à 80V jusqu'à ce que le bleu rentre dans le running gel, puis augmenter jusqu'à 100V; arrêter la migration quand le bleu est sorti du gel (env. 5 kDa).
- Transférer les gels sur une membrane nitrocellulose $0.45\mu$ (BIO-RAD ref: 1620115) pendant 1 h à 100V dans le tampon de transfert.
- Conserver les membranes humides au réfrigérateur, jusqu'à utilisation.

**MODE OPERATOIRE POUR L'UTILISATION DE L'ANTICORPS**

*POLYCLONAL MALI (Pab Mali).*

- Toutes les étapes d'incubation/lavage se font sur un agitateur à plateau de type Platform Shaker SRP6 (Stuart Sciences) à la vitesse de 20/25 REV/mn à température ambiante à $\cong$ 23°C.
- Les volumes de solution utilisés pour les étapes d'incubation/lavages sont de 20 ml et effectuées dans des boîtes de plastique de 112mm x 77 mm.
- La membrane est incubée pendant 1h avec la **solution de blocage**
- Incubation 1h avec le 1er anticorps **Polyclonal Mali** (anti RYMV) dilué au 1/1000ème dans la même solution de blocage (récupérer la solution, ajouter l'anticorps, agiter et remettre sur la membrane).
- Rinçages 6 x 5 mn dans le **TBS pH 7.5**
- Incubation 1h avec le 2ème anticorps conjugué **HRP- anti-lapin** dilué au 1/40000ème dans la solution de blocage neuve.
- Rinçages 6 x 5 mn dans le **TBS pH 7.5.**
- Mettre la membrane sur un film Saran, et verser de façon uniforme (la membrane doit être bien recouverte) la solution **West Pico** préparée en mélangeant à parts égales les 2 solutions (3 ml total par petite membrane).
- Attendre 5 mn (à la lumière), éliminer le substrat en excès, envelopper la membrane dans le Saran, puis mettre un film dessus (dans le noir) et exposer de 1 mn à 1 heure.
- Pour les hybridations aux pH 6,5 et pH 8,0, opérer de la même façon en utilisant un tampon **MES à pH 6.5** et un tampon **TAPS à pH 8.0** pour toutes les étapes d'hybridation et de lavage.

**MODE OPERATOIRE POUR L'UTILISATION DE L'ANTICORPS**

*MONOCLONAL E (Mab E).*

- Toutes les étapes d'incubation/lavage se font sur un agitateur à plateau de type Platform Shaker SRP6 (Stuart Sciences) à la vitesse de 20/25 REV/mn à température ambiante à $\cong$ 23°C.
- Les volumes de solution utilisés pour les étapes d'incubation/lavages sont de 20 ml et effectuées dans des boîtes de plastique de 112mm x 77 mm.
- La membrane est incubée pendant 1h avec la **solution de blocage**
- Incubation 1h avec le 1er anticorps **Monoclonal E** (épitope anti RYMV) dilué au **1/100ème** ou au **1/1000ème** dans la même solution de blocage (récupérer la solution, ajouter l'anticorps, agiter et remettre sur la membrane).
- Rinçages 6 x 5 mn dans le **TBS pH 7.5**
- Incubation 1h avec le 2ème anticorps conjugué H**RP- anti-souris** dilué au **1/40000ème** dans la solution de blocage neuve.
- Rinçages 6 x 5 mn dans le **TBS pH 7.5.**
- Mettre la membrane sur un film Saran, et versé de façon uniforme (la membrane doit être bien recouverte) la solution **West Pico** préparée en mélangeant à parts égales les 2 solutions (3 ml total par petite membrane).
- Attendre 5 mn (à la lumière), éliminer le substrat en excès, envelopper la membrane dans le Saran, puis mettre un film dessus (dans le noir) et exposer de 1 mn à 1 heure.
- Pour les hybridations aux pH 6,5 et pH 8,0, opérer de la même façon en utilisant un tampon **MES à pH 6,5** et un tampon **TAPS à pH 8,0** pour toutes les étapes d'hybridation et de lavage.

**SOLUTIONS:**

• **Tampon de charge 2X:**

| Concentration de la solution 2X | Quantité de produit pour 10 ml de sol. 2X |
| --- | --- |
| 100 mM Tris-HCl pH 6.8 | 1 ml de Tris-HCl 1M pH 6.8 |
| 200 mM DTT | 0.308 g |
| 4 % SDS | 2 ml SDS à 20 % |
| 0.2 % Bleu de bromophénol | 20 mg |
| 20% glycérol | 2 ml $H_2O$ q.s.p. 10 ml |

• **Tampon de migration 10X :**

| Concentration de la solution 10X | Quantité de produit pour 11 de sol, 10X |
| --- | --- |
| 250 mM Tris base | 30,285 g de Tris base |
| 2,5 M glycine | 187,67 g de glycine |

• **Tampon de transfert:**

| Tris base | 2.42 g |
| --- | --- |
| Glycine | 11.26 g |
| Méthanol | 100 ml |
| $H_2O$ | q.s.p. 1 litre. |

• **Solution de blocage Polyclonaux:** 3 % de Non-Fat Dry Milk BIO-RAD (ref: 170-6404) dans le tampon approprié.

• **Solution de blocage Monoclonaux:** 0.5% de B.S.A. dans le tampon approprié.

• **TBS pH 7.5:** 2.423g Tris-base (20mM)+ 3,146g NaCl (75mM) + 0.508g $MgCl_26H_2O$ (2, mM) + 0.5ml de NP-40 (0.05% Tergitol à *chauffer avant utilisation car non liquide à température ambiante)* + $H_2O$ q.s.p. 1000ml. Ajuster à pH 7,5 avec HCl.

• **MES pH 6.5:** 3.904g MES (20 mM) + 4,937g KCl (75 mM) + 0,508g $MgCl_2,6H_2O$ (2,5 mM) + 0.5ml de NP-40 (0.05% Tergitol *à chauffer avant utilisation car non liquide à température ambiante)* + $H_2O$ q.s.p. 1000ml. Ajuster à pH 6,5 avec NaOH ou HCl.

• **TAPS pH 8.0:** 4,866g TAPS (20 mM) + 4,937g KCl (75 mM) + 0,508g $MgCl_2,6H_2O$ (2,5 mM) + 0,5ml de NP-40 (0,05% Tergitol *à chauffer avant utilisation car non liquide à température ambiante)* + $H_2O$ q.s.p. 1000ml. Ajuster à pH 8,0 avec NaOH ou HCl.

• **Polyclonal Mali (Pab Mali):** solution d'anticorps polyclonal obtenue à partir de la particule virale entière. A diluer au 1/1000ème.

• **Monoclonal E (Mab E):** solution d'anticorps monoclonal obtenue à partir d'un épitope du RYMV. A diluer au 1/50ème ou au 1/1000ème.

• **Conjugué HRP anti-lapin.** "ImmunoPure® Goat Anti-Rabbit IgG, (H+L), Peroxydase Conjugated" (ref PIERCE: 31460) à 0,8 mg/ml après restauration dans $H_2O$. Diluer au 1/40000ème.

• **Conjugué HRP** anti-souris: "ImmunoPure® Goat Anti-Mouse IgG, (H+L), Peroxydase Conjugated" (ref PIERCE: 31430) à 0,8 mg/ml après restauration dans $H_2O$. Diluer au 1/40000ème.

• **West Pico:** "SuperSignal® West Pico Chemiluminescent Substrate" (ref PIERCE: 34080). Mélanger à parts égales la Lumino/Enhancer Solution et la Stable Peroxydase Solution (3ml total pour une petite membrane 8cm x 5cm). La solution ainsi préparée se conserve 24 heures et s'utilise à la lumière.

## Références bibliographiques

(1) Chen, X. et al., (1997), Development of a microsatellite framework map providing genome-wide coverage in rice (Oryza sativa L) Theor Appl Genet 95 : 553-567.

(2) Panaud, O. et al., (1996), Development of microsatellite markers and characterization of simple sequence length polymorphism (SSLP) in rice (Oryza sativa L) Mol Gen Genet 252 : 597-607.

(3) Wu K.S. et al., (1993), Abundance, polymorphism and genetic mapping of microsatellites in rice. Mol Gen Genet 241 : 225-235.

(4) Zabeau et al., (1993), Selective restriction fragment amplification : a general method for DNA fingerprinting. EP 92402629.7.

(5) Vos et al., (1995), AFLP, a new technique for DNA fingerprinting. Nucleic Acids Research 23: 4407-4414.

(6) Temnyck et al. (2000), Theor Appl Genet 100:697-712.

## Revendications

1. Méthode d'isolement de protéines impliquées dans la reconnaissance et le transport ciblé d'un virus pathogène circulant *via* les plasmodermes dans une plante, **caractérisée en ce qu'**on soumet des complexes virus/protéines obtenus après chromatographie d'un extrait de plante sensible infectée (voie a), ou des complexes obtenus à partir de virus purifiés et mis en contact avec les protéines d'une suspension cellulaire de plante sensible (voie b), à une électrophorèse et un Western Blot en utilisant un anticorps monoclonal anti-protéine de capside, et qu'on récupère les bandes non immunodétectées.

2. Méthode selon la revendication 1, **caractérisée en ce que** le virus est le virus de Rice Yellow Mottle Virus (RYMV) et qu'on récupère à partir des bandes non immunodétectées des protéines de 5, 24, 42, 49, 59, 66, 70, 77 et 210 kDa (voie a), telles qu'identifiées sur les figures 13B et 13C, ou des protéines de 24, 45, 51, 57, 63, 85 et au-delà de 120 kDa (voie b), telles qu'identifiées sur les figures 15A et 15B.

## Claims

1. Method for isolating proteins involved in the recognition and targeted transport of a pathogenic virus circulating via the plasmodesmata in a plant, wherein virus/proteins complexes obtained after chromatography of a sensitive infected plant extract (pathway a) or complexes obtained from purified virus and by contacting with proteins a cellular suspension of a sensitive plant (pathway b), are subjected to electrophoresis and Western Blot using a capsid anti-protein monoclonal antibody, and the non-immunodetected bands are collected.

2. The method of claim 1, wherein the virus is Rice Yellow Mottle Virus (RYMV) and proteins of 5, 24, 42, 49, 59, 66, 70, 77 and 210 kDa are collected from the non-immunodetected bands (pathway a), such as identified on Figures 13B and 13C, or proteins of 24, 45, 51, 57, 63, 85 and beyond 120 kDa (pathway b), such as identified on Figures 15A and 15B.

## Patentansprüche

1. Verfahren zur Isolierung von Proteinen, die an der Erkennung und dem gelenkten Transport eines pathogenen Virus, das über die Plasmodesmen in einer Pflanze zirkuliert, beteiligt sind, **dadurch gekennzeichnet, dass** man die nach der Chromatographie eines Extrakts einer infizierten empfindlichen Pflanze erhaltenen Virus/Protein-Komplexe (Weg a) oder Komplexe, die ausgehend von gereinigten und mit den Proteinen einer Zellsuspension einer empfindlichen Pflanze in Kontakt gebrachten Viren erhalten wurden (Weg b), einer Elektrophorese und einem Western Blotting unterzieht, wobei man einen monoklonalen Anti-Kapsidprotein-Antikörper verwendet und die nicht immunodetektierten Banden gewinnt.

2. Verfahren gemäß Anspruch 1, **dadurch gekennzeichnet, dass** es sich bei dem Virus um das Rice Yellow Mottle Virus (RYMV) handelt und aus nicht immunodetektierten Banden Proteine von 5, 24, 42, 49, 59, 66, 70, 77 und 210 kDa (Weg a), wie sie auf den Figuren 13b und 13c identifiziert sind, oder Proteine von 24, 45, 51, 57, 63, 85 und über 120 kDa (Weg b), wie sie auf den Figuren 15a und 15b identifiziert sind, gewonnen werden.

## Figure 1

## Figure 2

Figure 3

## Figure 4

## Figure 5

Figure 6

Figure 7

Figure 8

| ApaI | KpnI | PstI | ScaI | XbaI | HaeIII |
|------|------|------|------|------|--------|
| T A I G | T A I G | T A I G | T A I G | T A I G G | T A I G |

Figure 9

Figure 10

(a) : 183 F2 IR64 x Gigante (b) : 328 F2 IR64 x Gigante

III : Mouvement local, réplication
dans les feuilles systémiquement infectées

*Recherche des protéines impliquées*
*dans la replication et le mouvement local*

virus encapsidé

III

(décapsidation et réplication )

I : Mouvement local
dans la feuille inoculée

*Recherche des protéines impliquées*
*dans la réplication et le mouvement local*

virus encapsidé

complexe viral

I

II : Réplication, encapsidation,
stabilisation des particules dans les vacuoles
et mouvement vasculaire

*Recherche des protéines impliquées*
*dans la réplication, le transport sub-*
*cellulaire et le mouvement vasculaire*

Protéines hôtes cibles

FIGURE 11

Figure 12

injuln
tampon sculact   14040004.bio - 100.0µl
ph. 8-0

A

injuhon 170 µg de Rymv
pH: 8-5   20040002.bio - 100.0µl    Melode 1

B

Figure

Figure 12 suite

11050003.bio - 100.0µl

11050004.bio - 100.0µl

Figure 13A

Figure 13C

Figure 13B

f. B2 f. B3 f.B4 f.B9 f.B10 f.B11 f.B12 f.B13 f.B14 MWMW f.B14 f.B13 f.B12 f.B11 f.B10 f.B9 f.B4 f.B3 f. B2

f.B15 f.B16 f.B17 f.B18 f.B19 f.B20 f.B21 f.B22 f.B23 MW MW f.B23 f.B22 f.B21 f.B20 f.B19 f.B18 f.B17 f.B16 f.B15

Figure 14A

11050003.bio - 100.0µl

Figure 14C                 Figure 14B

Gel 13

f.A2 f.A3 f.A16 f.A17 f.A18 f.A28 f.A29 f.A30 f.B33 MW    MW f.B33 f.A30 f.A29 f.A28 f.A18 f.A17 f.A16 f.A3 f. A2

Figure 15A                                    Figure 15B

34.9 kDa

29.1 kDa

20.7 kDa

6.9 kDa

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 92402629 A
- DE 597821158
- DE 9907831

**Littérature non-brevet citée dans la description**

- **OPALKA et al.** *Proceedings of the National Academy of Sciences USA,* 1998, vol. 95, 3323-3328
- **CHEN, X. et al.** Development of a microsatellite framework map providing genome-wide coverage in rice (Oryza sativa L. *Theor Appl Genet,* 1997, vol. 95, 553-567
- **PANAUD, O. et al.** Development of microsatellite markers and characterization of simple sequence length polymorphism (SSLP) in rice (Oryza sativa L). *Mol Gen Genet,* 1996, vol. 252, 597-607
- **WU K.S. et al.** Abundance, polymorphism and genetic mapping of microsatellites in rice. *Mol Gen Genet,* 1993, vol. 241, 225-235
- **VOS et al.** AFLP, a new technique for DNA fingerprinting. *Nucleic Acids Research,* 1995, vol. 23, 4407-4414
- **TEMNYCK et al.** *Theor Appl Genet,* 2000, vol. 100, 697-712